(19) Europäisches Patentamt
European Patent Office
Office européen des brevets

(11) Veröffentlichungsnummer: **0 101 069**
**B1**

(12) # EUROPÄISCHE PATENTSCHRIFT

(45) Veröffentlichungstag der Patentschrift: **23.04.86**

(21) Anmeldenummer: **83107908.2**

(22) Anmeldetag: **10.08.83**

(51) Int. Cl.⁴: **C 07 C 103/28,** C 07 D 333/20, C 07 D 333/38, C 07 D 333/24, C 07 D 333/22, C 07 C 101/42, C 07 C 121/78, C 07 C 101/28, C 07 C 143/80, C 07 C 93/14

(54) **Neue Phenäthanolamine.**

(30) Priorität: **10.08.82 CH 4787/82**
**27.05.83 CH 2897/83**

(43) Veröffentlichungstag der Anmeldung:
**22.02.84 Patentblatt 84/8**

(45) Bekanntmachung des Hinweises auf die Patenterteilung:
**23.04.86 Patentblatt 86/17**

(84) Benannte Vertragsstaaten:
**AT BE CH DE FR GB IT LI LU NL SE**

(56) Entgegenhaltungen:
**EP - A - 0 007 204**
**DE - A - 1 543 672**
**FR - A - 2 211 251**

(73) Patentinhaber: **F. HOFFMANN-LA ROCHE & CO. Aktiengesellschaft, CH-4002 Basel (CH)**

(72) Erfinder: **Alig, Leo, Dr., Liebrütistrasse 32, CH-4303 Kaiseraugst (CH)**
Erfinder: **Müller, Marcel, Dr., Quellenweg 10, CH-4402 Frenkendorf (CH)**

(74) Vertreter: **Lederer, Franz, Dr. et al, Patentanwälte Dr. Franz Lederer Dipl.-Ing. Reiner F. Meyer-Roxlau Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 101 069 B1

**Beschreibung**

Die vorliegende Erfindung betrifft neue Phenäthanolamine, Verfahren zu ihrer Herstellung, neue Zwischenprodukte dazu und pharmazeutische Präparate auf der Basis dieser Verbindungen.

Die erfindungsgemässen Phenäthanolamine sind Verbindungen der Formel

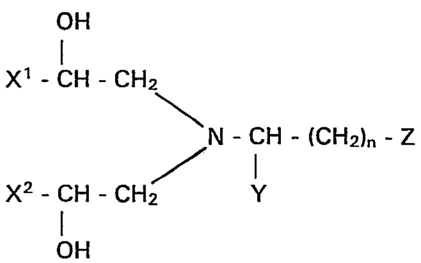

I

worin

n eine ganze Zahl von 1 bis 5;

$X^1$ Phenyl oder durch $R^1$, $R^2$ und $R^3$ mono-, di- oder tri-substituiertes Phenyl;

$X^2$ Wasserstoff, nieder-Alkyl, Phenyl oder durch $R^1$, $R^2$ und $R^3$ mono-, di- oder tri-substituiertes Phenyl;

$R^1$, $R^2$ und $R^3$ Wasserstoff, Halogen, Hydroxy, Benzyloxy, nieder-Alkyl, nieder-Alkoxy, Hydroxymethyl, Amino, Acylamino, nieder-Alkoxybenzylamino, Nitro, Carbamoyl, Trifluormethyl pder nieder-Alkylsulfonylmethyl;

Y Wasserstoff, nieder-Alkyl, Hydroxymethyl, Carboxy oder nieder-Alkoxycarbonyl;

Z ein Rest der Formel

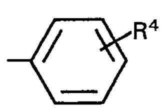

oder

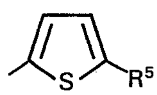

(Z)

$R^4$ und $R^5$ nieder-Alkanoyl, Carboxy, Cyano, Hydroxy-nieder-alkyl, Acyloxy oder einen Rest $-C(R^6)=C(R^7)COOR^8$, $-SO_2R^9$, $-C(O)R^9$ oder $-CH_2R^{10}$;

$R^6$, $R^7$ und $R^8$ Wasserstoff oder nieder-Alkyl;

$R^9$ Amino, mono-nieder-Alkylamino oder eine Gruppe R;

R di-nieder-Alkylamino, Piperidino, Morpholino, Thiamorpholino, Piperazino oder den Ätherrest eines niederaliphatischen, cycloaliphatischen oder araliphatischen Alkohols oder eines Phenols; und

$R^{10}$ eine Gruppe R darstellt und, falls $R^1$, $R^2$ und $R^3$ Wasserstoff, Halogen, Hydroxy, Benzyloxy, nieder-Alkyl, nieder-Alkoxy, Hydroxymethyl, Amino, nieder-Alkoxybenzylamino oder Trifluormethyl, und gleichzeitig Y Wasserstoff, nieder-Alkyl oder Hydroxymethyl sind, $R^{10}$ auch eine Gruppe Amino oder mono-nieder-Alkylamino darstellen kann,

sowie physiologisch verträgliche Salze davon.

Der hier verwendete Ausdruck «nieder» bezeichnet Reste mit 1-6 Kohlenstoffatomen, wobei Reste mit 1-4 Kohlenstoffatomen bevorzugt sind. Alkyl- und Alkoxygruppen können geradkettig oder verzweigt sein, Beispiele sind Methyl, Äthyl, Propyl, Isopropyl, n-Butyl und Isobutyl bzw. Methoxy, Äthoxy, Propoxy, Isopropoxy, Butoxy und Isobutoxy. Acylreste leiten sich von aliphatischen, araliphatischen oder aromatischen Carbonsäuren ab, z.B. nieder-Alkyncarbonsäuren, wie Ameisensäure, Essigsäure, Propionsäure und Buttersäure; oder Phenyl-nieder-alkancarbonsäuren, wie Phenylessigsäure; oder Benzoesäure. Beispiele von Alkoholresten R sind nieder-Alkoxygruppen; Cyclohexyloxy und Cyclopentyloxy; sowie Benzyloxy und substituierte Benzyloxygruppen wie p-Methoxybenzyloxy. Halogen ist vorzugsweise Chlor oder Brom.

Die Verbindungen der Formel I bilden mit Säuren Salze, die ebenfalls Gegenstand der Erfindung sind. Beispiele solcher Salze sind Salze mit physiologisch verträglichen Mineralsäuren, wie Salzsäure, Bromwasserstoffsäure, Schwefelsäure, Phosphorsäure; oder mit organischen Säuren, wie Methansulfonsäure, Essigsäure, Propionsäure, Zitronensäure, Bernsteinsäure, Äpfelsäure, Fumarsäure, Phenylessigsäure oder Salicylsäure. Carbonsäuren der Formel I können als Salze vorliegen. Beispiele für solche Salze sind Alkalimetall-, Erdalkalimetall-, Ammonium- und Alkylammoniumsalze, wie Na-, K-, Ca-, Trimethylammonium- und Äthanolammoniumsalze.

Die Verbindungen der Formel I enthalten ein oder mehrere asymmetrische Kohlenstoffatome und können somit als optisch aktive Enantiomere, als Diastereomere oder als Racemate vorliegen.

$X^1$ ist vorzugsweise Phenyl oder m-Trifluormethylphenyl, insbesondere Phenyl. $X^2$ ist vorzugsweise Phenyl, m-Trifluormethyl-phenyl, nieder-Alkyl oder Wasserstoff, insbesondere Phenyl. Y ist vorzugsweise Wasserstoff oder nieder-Alkyl, insbesondere Methyl. $R^4$ und $R^5$ sind vorzugsweise nieder-Alkanoyl, Carbamoyl, Sulfamoyl, nieder-Alkoxycarbonyl oder nieder-Alkylcarbamoyl, insbesondere nieder-Alkanoyl oder Carbamoyl.

Die Verbindungen der Formel I können erfindungsgemäss dadurch erhalten werden, dass man ein Epoxid der Formel

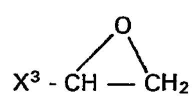

II

oder ein β-Ketohalogenid der Formel

$$X^3 - C(O) - CH_2 - Hal \qquad III$$

mit einem Amin der Formel

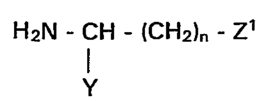

IV

oder

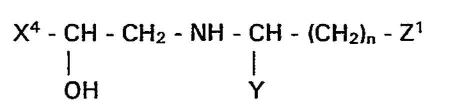

V

worin Hal Brom, Chlor oder Jod; eine der Gruppen $X^3$ und $X^4$ eine Gruppe $X^1$ und die andere eine Gruppe $X^2$ darstellt;

$Z^1$ einen Rest

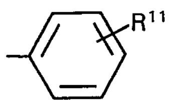
oder
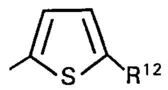
$(Z^1)$

$R^{11}$ und $R^{12}$ nieder-Alkanoyl, Carboxy, Cyano, Hydroxy-nieder-alkyl, Acyloxy oder einen Rest $-C(R^6)=C(R^7)COOR^8$, $-SO_2R^9$, $-C(O)R^9$ oder $-CH_2R$, und n, $X^1$, $X^2$, Y, R, $R^6$, $R^7$, $R^8$ und $R^9$ die bereits angegebene Bedeutung haben,
umsetzt, wobei $X^3$ eine Gruppe $X^1$ ist, falls man eine Verbindung der Formel II oder III mit einer solchen der Formel IV umsetzt; dass man eine in einer erhaltenen Verbindung vorhandene $X^1$-C(O)- bzw. $X^2$-C(O)-Gruppe zu einer $X^1$-CHOH- bzw. $X^2$-CHOH-Gruppe reduziert und dass man gewünschtenfalls einen in einer Gruppe $X^1$, $X^2$, Y oder $Z^1$ des Reaktionsproduktes enthaltenen reaktionsfähigen Substituenten funktionell abwandelt.

Die Umsetzung einer Verbindung der Formel II mit einer Verbindung der Formel IV oder V kann in für die Umsetzung von Epoxiden mit Aminen zu Aminoalkoholen an sich bekannter Weise vorgenommen werden. Zweckmässig werden die Reaktionspartner in einem geeigneten Lösungsmittel zusammengebracht und erwärmt. Als Lösungsmittel kommen inerte organische Lösungsmittel, z.B. Dimethylsulfoxid, Acetonitril oder Äther, wie Tetrahydrofuran oder Dioxan; oder Alkohole, wie Äthanol, in Betracht. Die Reaktionstemperatur ist nicht kritisch, zweckmässig arbeitet man bei Temperaturen zwischen 60°C und dem Siedepunkt des Reaktionsgemisches.

Die Umsetzung einer Verbindung der Formel III mit einer solchen der Formel IV oder V kann ebenfalls in an sich bekannter Weise, zweckmässig in Gegenwart eines Lösungsmittels, vorzugsweise eines aprotischen Lösungsmittels, wie einem halogenierten Kohlenwasserstoff, z.B. Chloroform, bei einer Temperatur bis zu 200°C durchgeführt werden.

Bei Umsetzung einer Verbindung III mit einer Verbindung IV bzw. V entstandene Ketogruppen $X^1$-C(O)- bzw. $X^2$-C(O)- werden in an sich bekannter Weise zu den sekundären Alkoholgruppen reduziert. Diese Reduktion kann unter den gleichen Bedingungen wie die weiter unten beschriebene Reduktion der Verbindungen VI-X durchgeführt werden, wobei die Reduktion mit einem komplexen Metallhydrid, insbesondere $NaBH_4$, auf Grund ihrer Selektivität bevorzugt ist.

In dem so erhaltenen Reaktionsprodukt, einer Verbindung der Formel I, in der Z einen wie oben definierten Rest $Z^1$ darstellt, kann ein reaktionsfähiger Substituent, insbesondere eine Gruppe $-C(R^6)=C(R^7)-COOR^8$, $-C(O)R^9$ oder $-SO_2R^9$, funktionell abgewandelt werden. Die Veresterung einer Carboxylgruppe kann in an sich bekannter Weise, z.B. mittels Alkylhalogeniden, wie Methyljodid, und einer Base vorgenommen werden. Die Verseifung einer Estergruppe wird zweckmässig unter alkalischen Bedingungen, z.B. mittels wässrig-alkoholischem Alkalihydroxid, z.B. wässrig-methanolischem Kaliumhydroxid, vorgenommen.

Eine Carbamoylgruppe kann durch Reduktion, z.B. mit komplexen Metallhydriden wie $LiAlH_4$, zur Aminomethylgruppe reduziert werden. In analoger Weise kann eine mono-nieder-alkylierte Carbamoylgruppe zur entsprechenden N-mono-nieder-alkylierten Aminomethylgruppe reduziert werden.

Gewisse Ausgangsstoffe der Formel V sind bekannt, z.B. aus den europäischen Patentanmeldungen 7204 A1, 21636 A1 und 6735 A1. Die Verbindungen der Formel V können dadurch erhalten werden, dass man

a) eine Verbindung der Formel

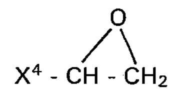
II-1

mit einer Verbindung der Formel IV umsetzt; oder

b) eine Verbindung einer der Formeln

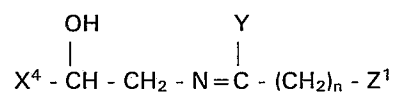
VI

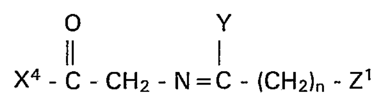
VII

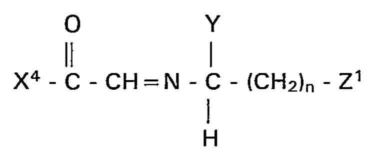
VIII

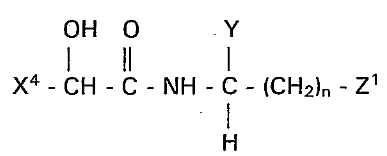
IX

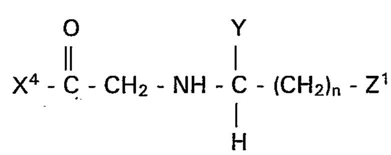
X

reduziert,
wobei in den vorstehenden Formeln die Reste $X^4$, $Z^1$, Y und n die früher angegebene Bedeutung haben.

Die Umsetzung einer Verbindung der Formel II-1 mit einer Verbindung der Formel IV kann in einem inerten organischen Lösungsmittel, zweckmässig einem protischen Lösungsmittel, wie einem niederen Alkanol, z.B. Äthanol, vorgenommen werden. Die Reaktionstemperatur ist nicht kritisch, sie kann zwischen Raumtemperatur und der Rückflusstemperatur des Reaktionsgemisches liegen.

Die Reduktion einer Verbindung der Formel VI kann durch katalytische Hydrierung, z.B. in Gegenwart von Edelmetallkatalysatoren, wie Pd- oder Pt-Katalysatoren oder durch Behandlung mit einem komplexen Metallhydrid, wie $NaBH_4$, vorgenommen

werden. Hierbei können die für derartige Reduktionen üblichen Reaktionsbedingungen angewandt werden. Die katalytische Hydrierung wird zweckmässig in einem inerten organischen Lösungsmittel, wie einem niederen Alkanol, z.B. Äthanol, bei Raumtemperatur oder schwach erhöhter Temperatur, z.B. bei 20-80°C durchgeführt. Die Reduktion mit einem komplexen Metallhydrid wird zweckmässig in einem niederen Alkanol, z.B. Methanol, bei Temperaturen von 20-30°C durchgeführt.

Die Verbindungen der Formeln VII, VIII, IX und X können mit einem komplexen Metallhydrid in Analogie zu den Verbindungen der Formel VI reduziert werden. Ein geeignetes komplexes Metallhydrid für die Reduktion der Verbindungen VII und VIII ist $NaBH_4$. Die Verbindungen IX werden zweckmässig mit $LiAlH_4$ reduziert.

Die Verbindungen der Formel I und ihre Salze können als Wirkstoffe in pharmazeutischen Präparaten zur Behandlung der Fettsucht und/oder des Diabetes mellitus, insbesondere des obesen Erwachsenendiabetikers verwendet werden. Im Tierexperiment wurde auf Verabreichung von Verbindungen der Formel I ein gesteigerter Katabolismus, vor allem der Fette, beobachtet. Weiterhin wurde beobachtet, dass die Verbindungen der Formel I die Bildung von braunem Fettgewebe bei Ratten und obes-hyperglykämischen Mäusen stimulieren. Bekanntlich wird Defekten des braunen Fettgewebes eine wesentliche Rolle bei der Entstehung der Fettsucht zugeschrieben. An obes-hyperglykämischen Mäusen haben die Verbindungen der Formel I einen ausgeprägten antidiabetischen Effekt, indem sie hypoglykämisch wirken und die Glykosurie vermindern. Die Verbindungen der Formel I zeigen nur eine geringe Wirkung auf Herztätigkeit und Kreislauf. Die Dosierung kann in Abhängigkeit von der Wirkungsstärke der einzelnen Verbindungen und den individuellen Bedürfnissen des Patienten 0,5-1000 mg, vorzugsweise 2-200 mg pro Tag für einen Erwachsenen betragen, wobei die Dosis als Einzeldosis oder in mehreren Dosen über den Tag verteilt verabreicht werden kann.

Ferner konnte mit den Verbindungen der Formel I im Tierexperiment eine Erhöhung des Körper-Proteingehaltes und eine Erniedrigung des Fettgehalts nachgewiesen werden. Die Verbindungen der Formel I führen demnach zu einer Erhöhung der mageren Körpermasse auf Kosten von Fett. Daher können die Verbindungen der Formel I zunächst in der Humanmedizin zur Behandlung von Zuständen, die mit erhöhtem Proteinabbau verbunden sind, z.B. bei Rekonvaleszenz nach Operation, verwendet werden. Dabei sind die Verabreichungsdosen die gleichen wie bei der Behandlung der Fettsucht und/oder des Diabetes mellitus.

Die Verbindungen der Formel I und ihre Salze können auch in der Ernährung von Masttieren, wie Rinder, Schweine, Schafe und Geflügel, Verwendung finden. Dabei können die Verabreichungsdosen und Verabreichungsformen die gleichen sein wie für Vitamine. Die Verbindungen der Formel I können auch als Futterzusatz in Dosen von 0,01-100 mg/kg je nach Substanz, Tierart und Alter eingesetzt werden.

Die pharmazeutischen Präparate enthalten den Wirkstoff zusammen mit einem verträglichen pharmazeutischen, organischen oder anorganischen Trägermaterial, wie z.B. Wasser, Gelatine, Gummi arabicum, Lactose, Stärke, Magnesiumstearat, Talk, pflanzliche Öle, Polyalkylenglykole, Vaseline und dergleichen. Die pharmazeutischen Präparate werden vorzugsweise oral, z.B. in Form von Tabletten, Kapseln, Pillen, Pulver, Granulaten, Lösungen, Sirupen, Suspensionen, Elixieren und dergleichen verabreicht. Die Verabreichung kann aber auch parenteral, z.B. in Form von sterilen Lösungen, Suspensionen oder Emulsionen, erfolgen. Die pharmazeutischen Präparate können sterilisiert sein und/oder Bestandteile enthalten, wie Konservierungsmittel, Stabilisatoren, Netzmittel, Emulgatoren, Salze, um den osmotischen Druck zu variieren, und Puffersubstanzen.

Die Aktivität der neuen Verbindungen der Formel I wird aus den nachstehenden Versuchsresultaten deutlich:

1) *Wirkung auf den Sauerstoffverbrauch*

Männliche Albinoratten im Gewicht von 160-180 g wurden nach 24 Stunden Fasten in Stoffwechselkäfige gesetzt. Die Käfige wurden mit konstant 6 Liter Raumluft/Minute, die bei einem Taupunkt von 11°C äquilibriert wurde, belüftet. Von der Abluft wurde nach erneuter Äquilibrierung während Perioden von jeweils 14 Minuten Proben gesammelt und der Sauerstoff- und $CO_2$-Gehalt analysiert. Nach einer Anpassungszeit von 4 Stunden erhielten die in Gruppen zu 6 aufgeteilten Tiere entweder Placebo (5% Gummi arabicum) oder die Testsubstanz (suspendiert in 5% Gummi arabicum) per os. Danach wurden 12 Stunden lang die Bestimmungen durchgeführt. In Tabelle I ist der Prozentsatz des gemittelten Sauerstoffverbrauchs nach Medikation während der ersten 3 Stunden und der gesamten Versuchsdauer (12 Stunden) vom Sauerstoffverbrauch der Anpassungsperiode angegeben, wobei entsprechende Korrekturen für Änderungen in der Placebo-Gruppe berücksichtigt wurden.

*Tabelle I*

| Verbindung hergestellt in Beispiel No. | Dosis µM/kg | $O_2$-Verbrauch % vom Wert der Vorperiode | |
|---|---|---|---|
| | | 1.-3. Stunde | 1.-12. Stunde |
| 1 | 100 | 156 | 115 |
| 2 | 300 | 143 | 116 |
| 3 | 30 | 127 | 106 |
| 4 | 100 | 175 | 127 |
| 6 | 30 | 140 | 109 |
| 7 | 10 | 125 | 108 |
| 8 | 30 | 184 | 133 |
| 9 | 30 | 124 | 108 |
| 10 | 30 | 130 | 112 |
| 11 | 3 | 116 | 106 |
| 12 | 10 | 190 | 139 |
| 13 | 30 | 146 | 125 |
| 14 | 100 | 182 | 132 |

*Tabelle I (Fortsetzung)*

| Verbindung hergestellt in Beispiel No. | Dosis µM/kg | $O_2$-Verbrauch % vom Wert der Vorperiode 1.-3. Stunde | 1.-12. Stunde |
|---|---|---|---|
| 15 | 10 | 125 | 109 |
| 16 | 30 | 127 | 103 |
| 17 | 30 | 172 | 123 |
| 18 | 1 | 146 | 114 |
| 19 | 100 | 148 | 123 |
| 20 | 100 | 149 | 112 |
| 21 | 30 | 130 | 107 |
| 22 | 10 | 113 | 115 |
| 23 | 100 | 121 | 110 |
| 24 | 30 | 154 | 111 |
| 25 | 3 | 155 | 116 |
| 26 | 100 | 143 | 113 |

2) *Katabole Wirkung auf die Lipide*

Gruppen von 4 männlichen Albinoratten im Gewicht von 320-360 g wurden ohne Zugang zu Futter in Stoffwechselkäfigen gehalten. Sauerstoffverbrauch und $CO_2$-Produktion wurde während 12 Stunden gemessen. Nach 4 Stunden erhielten die Tiere Placebo (5% Gummi arabicum) oder die Testsubstanz (suspendiert in Gummi arabicum) per os. In der Tabelle II ist die gemittelte Abnahme des respiratorischen Quotienten ($CO_2/O_2$) während 8 Stunden nach Verabreichung der Testsubstanz im Vergleich zu den letzten 3 Stunden vor Verabreichung der Testsubstanz angegeben. In den Placebo-Gruppen aufgetretene Änderungen wurden bei der Berechnung berücksichtigt.

*Tabelle II*

| Verbindung hergestellt in Beispiel No. | Dosis µM/kg | Änderung des respiratorischen Quotienten |
|---|---|---|
| 11 | 30 | —0,013 |
| 12 | 10 | —0,032 |

3) *Wirkung auf Harn- und Blutglucose und die Bildung von braunem Fettgewebe*

Weibliche hyperglykämische Fettmäuse wurden an eine auf 3 g/Tag/Tier begrenzte Futtermenge angepasst. Die Testverbindungen (suspendiert in 5% Gummi arabicum) oder Placebo (5% Gummi arabicum) wurden während 15 Tagen zweimal täglich oral verabreicht. Harn wurde während 6 Tagen wöchentlich gesammelt und Harnglucose bestimmt. Blutglucose und das Gewicht des interskapulären braunen Fettgewebes wurden am Versuchsende bestimmt.

Die Versuchsresultate sind in Tabelle III als Prozentsätze der Kontrollwerte angegeben.

*Tabelle III*

| Verbindung hergestellt in Beispiel No. | Dosis µM/kg pro Tag | Harnglucose 1. Woche | / 2. Woche | Blut-glucose | braunes Fett-gewebe |
|---|---|---|---|---|---|
| 12 | 60 | 35% | 4% | 50% | 224% |

4) *Wirkung auf Körpergehalt an Protein und Fett*

Weibliche hyperglykämische Fettmäuse wurden wie unter 3) beschrieben während 15 Tagen mit der Testverbindung behandelt. Nach Behandlungsende wurde die Karkass-Zusammensetzung bestimmt. Die Messresultate sind in Prozent des Karkassgewichts in Tabelle IV angegeben.

*Tabelle IV*

| Verbindung hergestellt in Beispiel No. | Dosis µM/kg/ Tag, oral | Protein-Gehalt Kontroll-Tiere | Protein-Gehalt Präparat-Tiere | Fettgehalt Kontroll-Tiere | Fettgehalt Präparat-Tiere |
|---|---|---|---|---|---|
| 12 | 60 | 8,15% | 9,35% | 59,9% | 53,7% |

Die folgenden Beispiele erläutern die Erfindung weiter.

*Beispiel 1*

2,68 g 5-[3-[[(S)-β-Hydroxyphenäthyl]amino]-propyl]-3-thiophencarbonsäureamid und 1,5 ml (R)--Phenyläthylenoxid wurden in 37 ml DMSO 24 Stunden bei 95°C gerührt. Das Reaktionsgemisch wurde auf Wasser gegossen und dreimal mit Methylenchlorid extrahiert. Die Methylenchloridlösungen wurden noch zweimal mit Wasser gewaschen, getrocknet und im Vakuum eingedampft. Chromatographie des Rückstandes an Kieselgel mit Äther-Methanol gab 1,48 g 5-[3-[[(R)-β-Hydroxyphenäthyl]-[(S)-β-hydroxyphenäthyl]amino]propyl]-2-thiophencarbonsäureamid, UV: $\varepsilon_{277}$ = 10780.

Das als Ausgangsmaterial verwendete 5-[3-[[(S)--β-Hydroxyphenäthyl]amino]propyl]-2-thiophencarbonsäureamid kann wie folgt hergestellt werden:

2-(p-Toluolsulfonyloxy)propylthiophen (J. Org.

Chem. 36, 1971, 2236) wurde mit Acetylchlorid und Aluminiumtrichlorid in Methylenchlorid zu 5-Acetyl--2-(p-toluolsulfonyloxy)propylthiophen umgesetzt. Mit Natriumazid in DMSO wurde daraus 5-(3-Azidopropyl)-2-thienyl-methylketon erhalten. Oxidation mit Hypobromid gab 5-(3-Azidopropyl)-2-thiophencarbonsäure, Smp. 71-72°. Umsetzen dieser Säure mit Thionylchlorid und anschliessende Behandlung mit konz. Ammoniak führte zum 5-(3-Azidopropyl)--2-thiophencarbonsäureamid, Smp. 85-87°. Daraus erhält man nach Behandlung mit Triphenylphosphin und Hydrolyse (J. Org. Chem. 40, 1975, 1659) 5-(3-Aminopropyl)-2-thiophencarbonsäureamid, Smp. 143,5-144° (aus Wasser).

5-(3-Aminopropyl)-2-thiophencarbonsäureamid wurde in DMSO bei 95° mit (S)-Phenyläthylenoxid erwärmt. Das Reaktionsgemisch wurde mit Wasser und Methylenchlorid verdünnt, die wässerige Phase wurde zweimal mit Methylenchlorid extrahiert; die Methylenchloridphasen wurden mit Wasser gewaschen, über Natriumsulfat getrocknet und im Vakuum eingedampft. Chromatographie des Rückstandes mit Methanol an Kieselgel gab 5-[3-[[(S)-β--Hydroxyphenäthyl]amino]propyl]-2-thiophencarbonsäureamid, Smp. 94-96°C, $[\alpha]_{589}$ = +19° (c = 0,1% in Dioxan), $\varepsilon_{256}$ = 8490, $\varepsilon_{275}$ = 10780.

*Beispiel 2*

5 g 5-(3-Aminopropyl)-2-thiophencarbonsäureamid und 3,4 ml (S)-Phenyläthylenoxid wurden in 68 ml DMSO 26 Stunden bei 95° gerührt. Das Reaktionsgemisch wurde auf 200 ml Wasser gegossen und dreimal mit 150 ml Methylenchlorid extrahiert. Die Methylenchloridlösungen wurden mit Wasser gewaschen, getrocknet und im Vakuum eingedampft. Chromatographie des Rückstandes an Kieselgel mit Äther-Methanol gab 2,85 g 5-[3-[Bis-(S)--β-hydroxyphenäthyl]amino]propyl]-2-thiophencarbonsäureamid, $[\alpha]_D$ = +76° (in Dioxan, c = 0,1%), UV: $\varepsilon_{277}$ = 10390.

*Beispiel 3*

750 mg 5-(3-Aminopropyl)-2-thiophencarbonsäureamid und 0,47 ml (±)-Phenyläthylenoxid wurden in 4,7 ml Äthanol 4 Stunden zum Sieden erhitzt. Nach dem Abdampfen des Lösungsmittels im Vakuum wurde der Rückstand mit Äther-Methanol an Kieselgel chromatographiert. Es wurden 400 mg 5--[3-[Bis-(β-hydroxyphenäthyl)amino]propyl]-2-thiophencarbonsäureamid erhalten. UV: $\varepsilon_{277}$ = 11250.

*Beispiel 4*

30 g 5-(3-Aminopropyl)-2-thiophencarbonsäureamid und 20,2 ml (R)-Phenyläthylenoxid wurden in 400 ml DMSO 24 Stunden bei 95° gerührt. Nach dem Abkühlen wurde das Reaktionsgemisch mit 1,3 l Wasser verdünnt und dreimal mit ca. 600 ml Methylenchlorid extrahiert. Die Methylenchloridlösungen wurden noch zweimal mit Wasser gewaschen, über Natriumsulfat getrocknet und im Vakuum eingedampft. Chromatographie des Rückstandes an Kieselgel mit Äther-Methanol gab 21 g 5--[3-[Bis-[(R)-β-hydroxyphenäthyl]amino]propyl]-2--thiophencarbonsäureamid, UV: $\varepsilon_{276}$ = 9900, $[\alpha]_D$ = —69° (in Dioxan, c = 0,1%).

*Beispiel 5*

Gemäss Beispiel 3 wurde aus 4-(3-Aminopropyl)-benzoesäure-methylester und (±)-Phenyläthylenoxid der p-[3-[Bis-(β-hydroxyphenäthyl)amino]propyl]benzoesäure-methylester als amorphe Substanz erhalten. $\varepsilon_{238}$ = 17520.

*Beispiel 6*

Gemäss Beispiel 3 wurden aus 4-(3-Aminopropyl)benzoesäureamid und (±)-Phenyläthylenoxid das p-[3-[Bis-(β-hydroxyphenäthyl)amino]propyl]-benzamid erhalten; amorph; $\varepsilon_{236}$ = 14270.

*Beispiel 7*

Gemäss Beispiel 3 wurde aus 4-(3-Aminopropyl)-benzoesäureamid und (R)-Phenyläthylenoxid das p-[3-Bis-[[(R)-β-hydroxyphenäthyl]amino]propyl]-benzamid erhalten. $\varepsilon_{234}$ = 14240.

*Beispiel 8*

Gemäss Beispiel 3 wurde aus (S)-1-Methyl-3-(4--amino-carbonylphenyl)propylamin und (R)-Styroloxid das p-[(S)-3-[Bis-[(R)-β-hydroxyphenäthyl)amino]-butyl]-benzamid als amorphe Substanz erhalten. $\varepsilon_{235}$ = 14130; $[\alpha]_D^{20}$ = —28° (c = 0,5 in Methanol).

*Beispiel 9*

Gemäss Beispiel 3 wurden aus 4-(3-Aminopropyl)benzolsulfonamid und R-Styroloxid das p-[3--[Bis-[(R)-β-hydroxyphenäthyl]amino]propyl]benzolsulfonamid als amorphe Substanz erhalten. $\varepsilon_{225}$ = 16820; $[\alpha]_D^{20}$ = —60° (c = 1,0 in Methanol).

Das als Ausgangsmaterial verwendete 4-(3-Aminopropyl)-benzolsulfonamid kann wie folgt hergestellt werden:

p-Aminosulfonylbenzaldehyd wurde mit Diäthyl--cyano-methyl-phosphonat(NaH in Tetrahydrofuran zu 1-Cyano-2-(4-aminosulfonylphenyl)-äthan) umgesetzt, welches in Methanol mit Raney-Cobalt als Katalysator zum 4-(3-Aminopropyl)-benzolsulfonamid hydriert wurde.

*Beispiel 10*

Gemäss Beispiel 3 wurde aus R-1-Methyl-3-(4--aminocarbonylphenyl)propylamin und (S)-Phenyläthylenoxid das p-[(R)-3-[Bis-[(S)-β-hydroxyphenäthyl]amino]butyl]benzamid als farblose, amorphe Substanz erhalten. $\varepsilon_{235}$ = 14230; $[\alpha]_D^{20}$ = +45° (c = 1,0 in Methanol).

*Beispiel 11*

Gemäss Beispiel 3 wurde aus R-1-Methyl-3-(4--aminosulfonylphenyl)propylamin und R-Styroloxid das p-[(R)-3-[Bis-[(R)-β-hydroxyphenäthyl]amino]-butyl]benzolsulfonamid als amorphe Substanz erhalten. $\varepsilon_{224}$ = 17410; $[\alpha]_D^{20}$ = —95° (c = 0,5 in Methanol).

*Beispiel 12*

Gemäss Beispiel 3 wurde aus R-Styroloxid und R-1-Methyl-3-(4-aminocarbonylphenyl)propylamin das p-[(R)-3-[Bis-[(R)-β-hydroxyphenäthyl]amino]-butyl]benzamid erhalten. $\varepsilon_{234}$ = 14490; $[\alpha]_D^{20}$ = —99° (c = 0,8 in Methanol).

*Beispiel 13*

Gemäss Beispiel 3 wurde aus R-Styroloxid und S-1-Methyl-3-(4-aminosulfonylphenyl)propylamin das p-[(S)-3-[Bis-[(R)-β-hydroxyphenäthyl]amino]-butyl]benzolsulfonamid als amorphe Substanz erhalten. $\varepsilon_{232}$ = 14820; $[\alpha]_D^{20}$ = —13° (c = 0,5 in Methanol).

Das als Ausgangsmaterial verwendete S-1-Methyl-3-(4-aminosulfonylphenyl)propylamin kann wie folgt hergestellt werden:

4-(4-Aminosulfonylphenyl)butanon-2 wurde mit S-(—)-α-Phenyläthylamin und p-Toluolsulfonsäure als Katalysator in Toluol unter Wasserabscheidung zur Schiff'schen Base S-N-(α-Methylbenzyl)-1-methyl-3-(4-aminosulfonylphenyl)-propylamin umgesetzt. Das Imin wurde in Methanol in Gegenwart von Raney-Nickel zu einem Gemisch der optischen Isomeren von N-(α-Methylbenzyl)-1-methyl-3-(4-aminosulfonylphenyl)propylamin hydriert. Das Amin wurde mit Oxalsäure in ein Gemisch der Oxalate übergeführt, aus dem durch zweimalige Kristallisation reines S-1-Methyl-3-(4-aminosulfonylphenyl)-propylamin-oxalat, Smp. 123-127°, $[\alpha]_D^{20}$ = —68° (c = 1,0 in Methanol) erhalten wurde. Hydrogenolyse dieser Substanz in Alkohol unter 4 bar $H_2$ bei 60°/24 Stunden lieferte reines S-1-Methyl-3-(4-aminosulfonylphenyl)propylamin.

*Beispiel 14*

Zu einer auf 0° abgekühlten Suspension von 1,0 g p-[3-[[(R)-β-Hydroxyphenäthyl]amino]propyl]-benzamid in 20 ml 90% Äthanol gibt man unter Rühren 5 ml Äthylenoxid. Nach 30 Minuten Rühren bei +5° war alles gelöst, worauf die Lösung noch 20 Stunden bei +5° gehalten wurde. Zur Aufarbeitung wurde das Lösungsmittel und überschüssiges Äthylenoxid im Vakuum eingedampft und der Rückstand über 100 g Kieselgel chromatographiert. Mit Essigester-Methanol (95:5) konnten 1,0 g reines, amorphes p-[3-[(2-Hydroxyäthyl)-[(R)-β-hydroxyphenäthyl]amino]propyl]benzamid eluiert werden. $[\alpha]_D^{20}$ = —44° (c = 0,5 in Methanol); $\varepsilon_{236}$ = 13700.

*Beispiel 15*

Gemäss Beispiel 3 wurde aus (S)-1-Methyl-3-(4-methyl-aminocarbonylphenyl)propylamin und R-Styroloxid das p-[(S)-3-[Bis-[(R)-α-hydroxyphenäthyl]amino]butyl]-N-methylbenzamid als amorphe Substanz erhalten. $[\alpha]_D^{20}$ = —22° (c = 1,0 in Methanol), $\varepsilon_{235}$ = 12800.

*Beispiel 16*

Gemäss Beispiel 14 wurde aus p-[3-[[(R)-β-Hydroxyphenäthyl]amino]propyl]benzamid und Propylenoxid (1,2-Epoxypropan) das p-[3-[[(R)-β-Hydroxyphenäthyl]-[(R,S)-2-hydroxypropyl]amino]propyl]benzamid als amorphe Substanz erhalten. $[\alpha]_D^{20}$ = —40° (c = 0,4 in Methanol).

*Beispiel 17*

Gemäss Beispiel 14 wurde aus p-[(S)-3-[[(R)-β-Hydroxyphenäthyl]amino]-butyl]benzamid und Äthylenoxid das p-[(S)-3-[(2-Hydroxyäthyl]-[(R)-β-hydroxyphenäthyl]amino]butyl]benzamid als Öl erhalten. $[\alpha]_D^{20}$ = —18° (c = 0,5 in Methanol).

*Beispiel 18*

Gemäss Beispiel 14 wurde aus p-[(R)-3-[[(R)-β-Hydroxyphenäthyl]amino]-butyl]benzamid und Äthylenoxid das p-[(S)-3-[(2-Hydroxyäthyl)-[(R)-β-hydroxyphenäthyl]amino]butyl]benzamid als farbloses Öl erhalten. $[\alpha]_D^{20}$ = —76° (c = 0,3 in Methanol).

*Beispiel 19*

850 mg 5-[3-[Bis-[(R)-β-hydroxyphenäthyl]amino]propyl]-2-thiophencarbonsäureamid und 300 mg $LiAlH_4$ wurden in 60 ml THF 1,5 Stunden am Rückfluss gekocht. Das Reaktionsgemisch wurde mit 2N Natronlauge vorsichtig zersetzt und dreimal mit Äther extrahiert. Die Ätherlösungen wurden mit Wasser neutral gewaschen, getrocknet und eingedampft. Chromatographie des Rückstandes an Kieselgel mit Methanol gab 650 mg 5-[3-[Bis-[(R)-β-hydroxyphenäthyl]amino]propyl]-2-amino-methyl-thiophen.

*Beispiel 20*

Gemäss Beispiel 14 wurde aus p-[3-[[(R)-β-Hydroxyphenäthyl]amino]propyl]benzolsulfonamid und Äthylenoxid das p-[3-[(2-Hydroxyäthyl)-[(R)-β-hydroxyphenäthyl]amino]propyl]benzolsulfonamid als farbloses Öl erhalten. $[\alpha]_D^{20}$ = —39° (c = 0,3 in Methanol); $\varepsilon_{224}$ = 14720.

Das als Ausgangsmaterial verwendete p-[3-[[(R)-β-Hydroxyphenäthyl]amino]propyl]benzolsulfonamid kann wie folgt hergestellt werden:

Eine Mischung von 20 g 4-(3-Aminopropyl)benzolsulfonamid, 16,8 g (R)-Styroloxyd und 500 ml Acetonitril wurde 40 Stunden unter Rückfluss erhitzt. Das Lösungsmittel wurde dann im Vakuum abgedampft und der Rückstand auf 1 kg Kieselgel chromatographiert. Mit Chloroform/n-Propanol/25% $NH_3$ (1600:100:4) konnten 8,3 g reines p-[3-[[(R)-β-Hydroxyphenäthyl]amino]propyl]-benzolsulfonamid vom Schmelzpunkt 165-166° (aus Acetonitril) isoliert werden, $[\alpha]_D^{20}$ = —13,4° (c = 1,0 in Methanol), $\varepsilon_{224}$ = 14710.

*Beispiel 21*

Gemäss Beispiel 3 wurde aus 4-(3-Aminopropyl)-benzonitril und R-Styroloxid das p-[3-[Bis-[(R)-β-hydroxyphenäthyl]amino]propyl]benzonitril als farbloses Öl erhalten. $[\alpha]_D^{20}$ = —57° (c = 0,4 in Methanol); $\varepsilon_{232}$ = 17570.

*Beispiel 22*

Gemäss Beispiel 3 wurde aus p-[(R)-2-Aminopropyl]-β-methyl-zimtsäuremethylester und 3-Trifluormethyl-styroloxid der p-[(R)-2-Bis-[(RS)-β-hydroxy-m-(trifluormethyl)phenäthyl]propyl]-β-methyl-zimtsäuremethylester erhalten. $[\alpha]_D^{20}$ = —53° (c = 0,2 in Methanol); $\varepsilon_{276}$ = 18500.

*Beispiel 23*

Gemäss Beispiel 3 wurde aus p-[(S)-2-Amino-propyl]-benzoesäure und R-Styroloxid die p-[(S)-2,2-Bis-[[(R)-β-hydroxyphenäthyl]amino]propyl]-benzoesäure erhalten. $[\alpha]_D^{20}$ = —3° (c = 0,5 in Methanol); $\varepsilon_{233}$ = 11700.

*Beispiel 24*

Gemäss Beispiel 3 wurde aus p-[(S)-2-Aminopropyl]-benzamid und Styroloxid das p-[(S)-2,2-Bis-[[(R)-β-hydroxyphenäthyl]amino]propyl]benzamid erhalten. $[\alpha]_D^{20}$ = —1° (c = 0,8 in Methanol); $\varepsilon_{226}$ = 11700.

*Beispiel 25*

In Analogie zu Beispiel 4 wurde aus (R,S)-5-(3-Aminobutyl)-2-thiophencarbonsäureamid und (R)-Phenyläthylenoxid das 5-[(RS)-3-[Bis-[(R)-β-hydroxyphenäthyl]amino]butyl-2-thiophencarbonsäureamid erhalten. $[\alpha]_D$ = —90° (0,1% in Dioxan); UV: $\varepsilon_{277}$ = 11100, $\varepsilon_{255}$ = 8700.

Das als Ausgangsmaterial verwendete (R,S)-5-(3-Aminobutyl)-2-thiophencarbonsäureamid kann wie folgt hergestellt werden:

4-(5-Acetyl-2-thienyl)-2-butanon (Tetrahedron 35, 1979, 329) wurde mit Äthylenglykol, o-Ameisensäuretriäthylester und p-Toluolsulfonsäure in Methylenchlorid selektiv zu Methyl 5-[2-(2-methyl-1,3-dioxolan-2-yl)äthyl]-2-thienylketon umgesetzt. Oxidation mit Natriumhypobromit und anschliessende Hydrolyse gab 5-(3-Oxobutyl)-2-thiophencarbonsäure. Mit $NaBH_4$ wurde daraus 5-(3-Hydroxybutyl)-2-thiophencarbonsäure erhalten, welche in Dimethylacetamid mit Methyljodid und Natriumbicarbonat in den Methylester übergeführt wurde. Behandlung mit p-Toluolsulfochlorid in Pyridin und Reaktion mit Natriumazid in DMSP gab 5-(3-Azidobutyl)-2-thiophencarbonsäuremethylester, aus welchem durch Verseifung die entsprechende Säure erhalten wurde. Mit Thionylchlorid wurde deren Säurechlorid hergestellt, aus dem mit konz. Ammoniak in Äther 5-(3-Azidobutyl)-2-thiophencarbonsäureamid erhalten wurde. Reduktion der Azidogruppe mit Triphenylphosphin und anschliessende Hydrolyse gab (R,S)-5-(3-Aminobutyl)-2-thiophencarbonsäureamid; Smp. 65-75°, $\varepsilon_{256}$ = 7780, $\varepsilon_{275}$ = 9900.

*Beispiel 26*

Ebenso wie in Beispiel 25 erhielt man aus p-(2-Aminoäthyl)benzamid und (R)-Phenyläthylenoxid das p-[2-[Bis-[(R)-β-hydroxyphenäthyl]amino]äthyl]benzamid. $[\alpha]_D$ = —59° (0,1% in Dioxan). UV: $\varepsilon_{234}$ = 13500.

Das als Ausgangsmaterial verwendete p-(2-Aminoäthyl)-benzamid kann wie folgt hergestellt werden:

p-(2-Bromäthyl)-acetophenon (J.A.C.S. 62, 1940, 1435) wurde mit Natriumazid in DMSP zu p-(2-Azidoäthyl)acetophenon umgesetzt. Oxidation mit Natriumhypobromid gab p-(2-Azidoäthyl)benzoesäure (Smp. 130-131°, aus Aceton-Hexan), die mit Thionylchlorid in das entsprechende Säurechlorid und anschliessend mit Ammoniak ins p-(2-Azidoäthyl)-benzamid überging. Behandlung mit Triphenylphosphin und Hydrolyse gab p-(2-Aminoäthyl)benzamid, Smp. 132-133° (aus Äthanol).

*Beispiel 27*

In Analogie zu Beispiel 4 wurde aus (R)-Phenyläthylenoxid und 5-(2-Aminoäthyl)-2-thiophencarbonsäureamid das 5-[2-[Bis-[(R)-β-hydroxyphenäthyl]amino]äthyl]-2-thiophencarbonsäureamid erhalten, $[\alpha]_D^{20}$ = —61° (0,1% in Dioxan), $\varepsilon_{277}$ = 10560.

Das als Ausgangsmaterial verwendete 5-(2-Aminoäthyl)-2-thiophencarbonsäureamid kann wie folgt hergestellt werden:

2-(2-Thienyl)äthyl-p-toluolsulfonat (J.A.C.S. 95, 1973, 1247), Acetylchlorid und Aluminiumchlorid wurden in Methylenchlorid zu 2-[(5-Acetyl-2-thienyl)-äthyl]-p-toluolsulfonat umgesetzt (Smp. 111-112°, aus Äthanol). Dieses wurde mit Natriumazid in DMSO in das 5-(2-Azidoäthyl)-2-thienylmethylketon übergeführt. Oxidation mit Natriumhypobromit lieferte die 5-(2-Azidoäthyl)-2-thiophencarbonsäure vom Smp. 53-55°, die mit Thionylchlorid das entsprechende Säurechlorid ergab, welches mit Ammoniak zum 5-(2-Azidoäthyl)-2-thiophencarbonsäureamid umgesetzt wurde (Smp. 104-105°, aus Äthanol). Reaktion mit Triphenylphosphin und Hydrolyse (J. Org. Chem. 40, 1975, 1659) gab 5-(2-Aminoäthyl)-2-thiophencarbonsäureamid, Smp. 134-136°, aus Acetonitril.

*Beispiel 28*

In Analogie zu Beispiel 4 wurde aus (R)-Phenyläthylenoxid und 5-[(RS)-2-Aminopropyl]-2-thiophencarbonsäureamid das 5-[(RS)-2-[Bis[(R)-β-hydroxyphenäthyl]amino]propyl]-2-thiophencarbonsäureamid hergestellt, $[\alpha]_D^{20}$ = —58° (c = 0,1% in Dioxan), $\varepsilon_{279}$ = 8450.

Das als Ausgangsmaterial verwendete 5-[(RS)-2-Aminopropyl]-2-thiophencarbonsäureamid kann wie folgt hergestellt werden:

α-Methyl-2-thiophenäthanol (J.A.C.S. 64, 1942, 477), Acetylchlorid und Aluminiumchlorid wurden in Methylenchlorid zu (RS)-2-(5-Acetyl-2-thienyl)-1-methyläthylacetat umgesetzt. Mit Natronlauge in Methanol wurde dieses zu 5-[(RS)-2-Hydroxypropyl]-2-thienylmethylketon verseift und anschliessend mit p-Toluolsulfochlorid zu (RS)-2-(5-Acetyl-2-thienyl)-1-methyläthyl-p-toluolsulfonat umgesetzt, Smp. 101-103°. Mit Natriumazid in DMSO wurde daraus 5-[(RS)-2-Azidopropyl]-2-thienylmethylketon erhalten, welches mit Brom in Natronlauge zur 5-[(RS)-2-Azidopropyl]-2-thiophencarbonsäure oxidiert wurde. Mit $SOCl_2$ wurde daraus das entsprechende Säurechlorid, und aus diesem mit Ammoniak 5-[(RS)-2-Azidopropyl]-2-thiophencarbonsäureamid hergestellt, Smp. 79-80°, aus Äther. Behandlung mit Triphenylphosphin und Hydrolyse gab 5-[(RS)-2-Aminopropyl]-2-thiophencarbonsäureamid, Smp. 91-92° aus Acetonitril.

*Beispiel 29*

Aus (RS)-5-(3-Aminobutyl)-2-thiophencarbonsäureamid und m-(Trifluormethyl)phenyläthylenoxid wurde in Analogie zu Beispiel 4 das 5-[(RS)-3-[Bis-[(RS)-β-hydroxy-m-(trifluormethyl)-phenäthyl]amino]butyl]-2-thiophencarbonsäureamid hergestellt, $\varepsilon_{272}$ = 11840.

*Beispiel 30*

5 g (E)-5-[(RS)-2-Aminopropyl]-β-methyl-2-thiophenacrylsäureäthylester und 3,7 g m-(Trifluormethyl)phenyl-äthylenoxid wurden in 50 ml DMSO 22 Stunden bei 90° gerührt. Es wurden nochmals

1,23 g (Trifluormethyl)phenyläthylenoxid zugegeben und es wurde weitere 19 Stunden auf 90° erwärmt. Die Aufarbeitung erfolgte analog zu Beispiel 4. Chromatographie an Kieselgel gab (E)-5-[(RS)-2--[bis-[(RS)-β-hydroxy-m-(trifluormethyl)phenäthyl]-amino]propyl]-β-methyl-2-thiophenacrylsäureäthylester, $\varepsilon_{323}$ = 17310.

Das als Ausgangsmaterial verwendete (E)-5-[(RS)--2-Aminopropyl]-β-methyl-2-thiophenacrylsäure-äthylester kann wie folgt hergestellt werden:

5-[(RS)-2-Hydroxypropyl]-2-thienylmethylketon und Triäthylphosphonoacetat wurden in Alkohol in Gegenwart von Natriumäthylat zu (E)-5-[(RS)-2--Hydroxypropyl]-β-methyl-2-thiophenacrylsäure-äthylester umgesetzt. Mit p-Toluolsulfochlorid wurde daraus der (E)-β-Methyl-5-[(RS)-2-[(p-toluolsulfonyl)oxy]propyl]-2-thiophenacrylsäureäthylester erhalten (Smp. 121°, aus Methylenchlorid-Alkohol). Reaktion mit Natriumazid in DMSO gab den (E)-5--[(RS)-2-Azidopropyl]-β-methyl-2-thiophenacrylsäureäthylester. Reduktion mit Triphenylphosphin und Hydrolyse führte zum (E)-5-[(RS)-2-Aminopropyl]-β-methyl-2-thiophenacrylsäureäthylester, $\varepsilon_{320}$ = 17970.

*Beispiel 31*

In Analogie zu Beispiel 30 wurden aus (E)-5--[(RS)-3-Aminobutyl]-β-methyl-2-thiophenacrylsäureäthylester und (R)-Phenyläthylenoxid

(E)-5-[(R)-3-[Bis[(R)-β-Hydroxyphenäthyl]amino]-butyl]-β-methyl-2-thiophenacrylsäureäthylester, $[\alpha]_D^{20}$ = —126° (c = 0,1% in Dioxan), $\varepsilon_{324}$ = 19030, und

(E)-5-[(S)-3-[Bis[(R)-β-Hydroxyphenäthyl]amino]-butyl]-β-methyl-2-thiophenacrylsäureäthylester, $[\alpha]_D^{20}$ = —40° (c = 0,1% in Dioxan), $\varepsilon_{323}$ = 18050 erhalten.

Das als Ausgangsmaterial verwendete (E)-5--[(RS)-3-Aminobutyl]-β-methyl-2-thiophenacrylsäureäthylester kann wie folgt hergestellt werden:

(RS)-4-(2-Thienyl)-2-butanol (Coll. Czech. Chem. Comm. 41, 1976, 479), Acetylchlorid und Aluminiumchlorid wurden in Methylenchlorid zu (RS)-3--(5-Acetyl-2-thienyl)-1-methylpropylacetat umgesetzt. Mit NaOH in Methanol wurde dieses zu (RS)--5-(3-Hydroxybutyl)-2-thienylmethylketon verseift. Dieses reagierte in Alkohol in Gegenwart von Natriumalkoholat mit Triäthylphosphonoacetat zu (E)--5-[(RS)-3-Hydroxybutyl]-β-methyl-2-thiophenacrylsäureäthylester. Umsetzen mit p-Toluolsulfochlorid und anschliessende Behandlung mit Natriumazid gab (E)-5-[(RS)-3-Azidobutyl]-β-methyl-2-thiophenacrylsäureäthylester. Daraus wurde durch Reduktion mit Triphenylphosphin und anschliessende Hydrolyse (E)-5-[(RS)-3-Aminobutyl]-β-methyl-2--thiophenacrylsäureäthylester erhalten, $\varepsilon_{320}$ = 17465.

*Beispiel 32*

In Analogie zu Beispiel 4 wurde aus m-(Trifluormethyl)-phenyläthylenoxid und p-(2-Aminoäthyl)--benzamid das p-[2-[Bis[(RS)-β-hydroxy-m-(trifluormethyl)phenäthyl]amino]äthyl]benzamid hergestellt, $\varepsilon_{232}$ = 14200.

*Beispiel 33*

In Analogie zu Beispiel 4 wurde aus (R)-Phenyläthylenoxid und 2-Acetyl-5-[(RS)-2-aminopropyl]-thiophen das 5-[(RS)-2-[Bis[(R)-β-hydroxyphenäthyl]amino]propyl]-2-thienyl-methylketon synthetisiert, $[\alpha]_D^{20}$ = —46° (c = 0,1% in Methanol), $\varepsilon_{298}$ = 10400, $\varepsilon_{263}$ = 7250.

Das als Ausgangsmaterial verwendete 2-Acetyl--5-[(RS)-2-aminopropyl]thiophen kann dadurch hergestellt werden, dass man das 5-[(RS)-2-Azidopropyl]-2-thienylmethylketon mit Triphenylphosphin umsetzt und anschliessend mit wässerigem Ammoniak hydrolysiert.

*Beispiel 34*

In Analogie zu Beispiel 30 wurde aus (E)-5-[(RS)--2-Aminopropyl]-β-methyl-2-thiophenacrylsäure-äthylester und (R)-Phenyläthylenoxid

(E)-5-[(R)-2-[Bis[(R)-β-hydroxyphenäthyl]amino]-propyl]-β-methyl-2-thiophenacrylsäureäthylester, $[\alpha]_D^{20}$ = —76° (c = 0,1% in Methanol), $\varepsilon_{324}$ = 18690, und

(E)-5-[(S)-2-[Bis[(R)-β-hydroxyphenäthyl]amino]-propyl]-β-methyl-2-thiophenacrylsäureäthylester, $[\alpha]_D^{20}$ = +18° (c = 0,1% in Methanol), $\varepsilon_{325}$ = 18250 erhalten.

*Beispiel 35*

In Analogie zu Beispiel 30 wurden aus 5-[(RS)--3-Aminobutyl]-2-thiophencarbonsäuremethylester und (R)-Phenyläthylenoxid

5-[(R)-3-[Bis[(R)-β-hydroxyphenäthyl]amino]butyl]-2-thiophencarbonsäuremethylester, $[\alpha]_D^{20}$ = —103° (c = 0,1% in MeOH), $\varepsilon_{279}$ = 13180, $\varepsilon_{255}$ = 9540, und

5-[(S)-3-[Bis[(R)-β-hydroxyphenäthyl]amino]butyl]-2-thiophencarbonsäuremethylester, $[\alpha]_D^{20}$ = —18° (c = 0,1% in MeOH), $\varepsilon_{279}$ = 12150, $\varepsilon_{255}$ = 8914 erhalten.

Das als Ausgangsmaterial verwendete 5-[(RS)--3-Aminobutyl]-2-thiophencarbonsäuremethylester kann dadurch hergestellt werden, dass man den 5--(3-Azidobutyl)-2-thiophencarbonsäuremethylester mit Triphenylphosphin in Pyridin umsetzt und mit konz. Ammoniak zum 5-[(RS)-3-Aminobutyl]-2--thiophencarbonsäuremethylester, $\varepsilon_{278}$ = 11280, $\varepsilon_{254}$ = 8760 hydrolysiert.

*Beispiel 36*

Gemäss Beispiel 2 wurde aus p-(3-Aminopropyl)--benzoesäuremethylester und (R)-Styroloxid der p--[Bis[(R)-Hydroxyphenäthyl]amino]propyl]-benzoesäuremethylester als amorphe Substanz erhalten. $[\alpha]_D^{20}$ = —60° (c = 1,0% in Methanol), $\varepsilon_{238}$ = 13770.

*Beispiel 37*

Gemäss Beispiel 2 wurde aus p-[(R)-2-Aminopropyl]-benzamid und (R)-Styroloxid das p-[(R)-2-[Bis-[(R)-β-Hydroxyphenäthyl]amino]propyl]-benzamid in amorpher Form erhalten. $[\alpha]_D^{20}$ = —102° (c = 0,71% in Methanol), $\varepsilon_{232}$ = 13780.

*Beispiel 38*

Gemäss Beispiel 2 wurde aus R-1-Methyl-3-(4--aminocarbonylphenyl)-propylamin und 3-Trifluormethylstyroloxid das p-[(R)-3-[Bis[(RS)-β-Hydroxy--m-(trifluormethyl)phenäthyl]amino]butyl]benzamid als amorphe Substanz erhalten. $[\alpha]_D^{20} = -20°$ (c = 1,0% in Methanol), $\varepsilon_{235} = 14990$.

*Beispiel 39*

Gemäss Beispiel 2 wurde aus p-[(R)-2-Aminopropyl]-benzoesäuremethylester und Styroloxid der p--[2-[bis[(R)-Hydroxyphenäthyl]amino]propyl]-benzoesäuremethylester erhalten (amorph). $[\alpha]_D^{20} = -91°$ (c = 0,6% in Methanol), $\varepsilon_{237} = 15720$.

*Beispiel 40*

Eine Mischung von 13,6 g 4-Amino-3,5-dichlorphenacylbromid und 4,6 g (R)-1-Methyl-3-(4-aminocarbonylphenyl)-propylamin in 100 ml Chloroform wurde 3 Stunden auf 50° erwärmt. Die Reaktionsmischung wurde im Vakuum eingedampft, der Rückstand in 200 ml Methanol und 70 ml Wasser gelöst und unter Eiskühlung und Rühren wurde eine Lösung von 3,0 g $NaBH_4$ in 20 ml Wasser so zugetropft, dass die Temperatur nicht über 20° stieg. Nach beendigter Zugabe wurde noch 2 Stunden bei 5-10° gerührt, dann auf Eiswasser gegossen und mit Methylenchlorid extrahiert. Das aus dem Methylenchlorid-Extrakt isolierte Material wurde auf Silicagel chromatographiert. Mit Chloroform/n-Propanol/ 25% $NH_3$ (1000:10:1) wurde reines p-[(R)-3-[Bis-[(RS)-4-amino-3,5-dichlor-β-hydroxyphenäthyl]-amino]butyl]benzamid in amorpher Form erhalten. $[\alpha]_D^{20} = -46°$ (c = 1,0% in Methanol), $\varepsilon_{242} = 31080$, $\varepsilon_{302} = 7250$.

*Beispiel 41*

Es werden in üblicher Weise Tabletten folgender Zusammensetzung hergestellt:

| | |
|---|---|
| Wirkstoff der Formel I, z.B. das p-[(R)-3-[Bis-[(R)-β-hydroxyphenäthyl]-amino]butyl]benzamid | 250 mg |
| Lactose | 200 mg |
| Maisstärke | 300 mg |
| Maisstärkepaste | 50 mg |
| Calciumstearat | 5 mg |
| Dicalciumphosphat | 45 mg |

*Beispiel 42*

216 mg p-[(R)-3-[Bis-[(R)-β-hydroxyphenäthyl]-amino]butyl]benzamid wurde in 2 ml Methanol gelöst und mit 58 mg Fumarsäure versetzt. Die Lösung wurde dann mit 10 ml Äther versetzt. Es schieden sich 210 mg kristallines p-[(R)-3-[Bis-[(R)-β-hydroxyphenäthyl]amino]butyl]benzamid-fumarat ab, die nach Umkristallisation aus Acetonitril bei 92-95° schmolzen; $[\alpha]_D^{20} = -81°$ (c = 1,0% in Methanol). $\varepsilon_{234} = 17400$.

*Beispiel 43*

In zu Beispiel 42 analoger Weise wurde das p--[(R)-3-[Bis-[(R)-β-hydroxyphenäthyl]amino]butyl]-benzamid-maleat erhalten, Smp. 127-128° (in Methanol-Äther) $[\alpha]_D^{20} = -78°$ (c = 0,8% in Methanol), $\varepsilon_{232} = 16900$.

**Patentansprüche für die Vertragsstaaten:**
BE, CH, DE, FR, GB, IT, LI, LU, NL, SE

1. Verbindungen der Formel

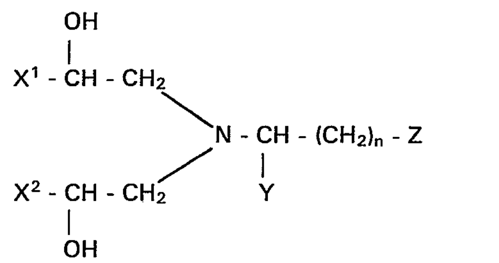

I

worin

n eine ganze Zahl von 1 bis 5;

$X^1$ Phenyl oder durch $R^1$, $R^2$ und $R^3$ mono-, di- oder tri-substituiertes Phenyl;

$X^2$ Wasserstoff, nieder-Alkyl, Phenyl oder durch $R^1$, $R^2$ und $R^3$ mono-, di- oder tri-substituiertes Phenyl;

$R^1$, $R^2$ und $R^3$ Wasserstoff, Halogen, Hydroxy, Benzyloxy, nieder-Alkyl, nieder-Alkoxy, Hydroxymethyl, Amino, Acylamino, nieder-Alkoxybenzylamino, Nitro, Carbamoyl, Trifluormethyl oder nieder-Alkylsulfonylmethyl;

Y Wasserstoff, nieder-Alkyl, Hydroxymethyl, Carboxy oder nieder-Alkoxycarbonyl;

Z ein Rest der Formel

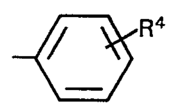

oder

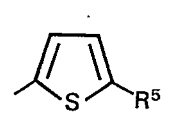

(Z)

$R^4$ und $R^5$ nieder-Alkanoyl, Carboxy, Cyano, Hydroxy-nieder-alkyl, Acyloxy oder einen Rest $-C(R^6)=C(R^7)COOR^8$, $-SO_2R^9$, $-C(O)R^9$ oder $-CH_2R^{10}$;

$R^6$, $R^7$ und $R^8$ Wasserstoff oder nieder-Alkyl;

$R^9$ Amino, mono-nieder-Alkylamino oder eine Gruppe R;

R di-nieder-Alkylamino, Piperidino, Morpholino, Thiamorpholino, Piperazino oder den Ätherrest eines niederaliphatischen, cycloaliphatischen oder araliphatischen Alkohols oder eines Phenols; und

$R^{10}$ eine Gruppe R darstellt und, falls $R^1$, $R^2$ und $R^3$ Wasserstoff, Halogen, Hydroxy, Benzyloxy, nieder-Alkyl, nieder-Alkoxy, Hydroxymethyl, Amino, nieder-Alkoxybenzylamino oder Trifluormethyl, und gleichzeitig Y Wasserstoff, nieder-Alkyl oder Hydroxymethyl sind, $R^{10}$ auch eine Gruppe Amino oder mono-nieder-Alkylamino darstellen kann,

sowie physiologisch verträgliche Salze davon.

2. Verbindungen gemäss Anspruch 1, in denen $R^1$ Wasserstoff, $R^2$ und $R^3$ Wasserstoff, Halogen, Hydroxy, Benzyloxy, nieder-Alkyl, nieder-Alkoxy, Hydroxymethyl, Amino, Acylamino, nieder-Alkylbenzylamino, Nitro, Trifluormethyl oder nieder-Alkylsulfonylmethyl; $R^4$ und $R^5$ Carboxy, Cyano, Hydroxy-nieder-alkyl, Acyloxy oder einen Rest $-C(R^6)=C(R^7)COOR^8$, $-SO_2R^9$, $-C(O)R^9$ oder $-CH_2R^{10}$ sind.

3. Verbindungen gemäss Anspruch 1 oder 2, in denen n 1 oder 2, insbesondere 2, ist.

4. Verbindungen gemäss Anspruch 1, 2 oder 3, in denen $X^1$ Phenyl oder m-Trifluormethyl-phenyl, insbesondere Phenyl, ist.

5. Verbindungen gemäss den Ansprüchen 1-4, in denen $X^2$ Phenyl, m-Trifluormethyl-phenyl, nieder-Alkyl oder Wasserstoff, insbesondere Phenyl, ist.

6. Verbindungen gemäss den Ansprüchen 1-5, in denen Y Wasserstoff oder nieder-Alkyl, insbesondere Methyl, ist.

7. Verbindungen gemäss den Ansprüchen 1 oder 3-6, in denen $R^4$ und $R^5$ nieder-Alkanoyl, Carbamoyl, Sulfamoyl, nieder-Alkoxycarbonyl oder nieder-Alkylcarbamoyl, insbesondere nieder-Alkanoyl oder Carbamoyl, ist.

8. Verbindungen gemäss den Ansprüchen 1 oder 3-7, in denen n 1 oder 2, insbesondere 2; $X^1$ Phenyl oder m-Trifluormethylphenyl, insbesondere Phenyl; $X^2$ Phenyl, m-Trifluormethyl-phenyl, nieder-Alkyl oder Wasserstoff, insbesondere Phenyl; Y Wasserstoff oder nieder-Alkyl, insbesondere Methyl; $R^4$ und $R^5$ nieder-Alkanoyl, Carbamoyl, Sulfamoyl, nieder-Alkoxycarbonyl, oder nieder-Alkylcarbamoyl, insbesondere nieder-Alkanoyl oder Carbamoyl, sind.

9. p-[(R)-3-[Bis-[(R)-β-hydroxyphenäthyl]amino]-butyl]benzamid.

10. Verbindungen der Formel I und physiologisch verträgliche Salze davon als Mittel zur Behandlung der Fettsucht, des Diabetes mellitus und von Zuständen, die mit erhöhtem Proteinabbau verbunden sind, bzw. als Futterzusatz für Masttiere.

11. Pharmazeutische Präparate, gekennzeichnet durch einen Gehalt an einer Verbindung der Formel I oder an einem physiologisch verträglichen Salz davon.

12. Verfahren zur Herstellung von Verbindungen der Formel I und von physiologisch verträglichen Salzen davon, dadurch gekennzeichnet, dass man ein Epoxid der Formel

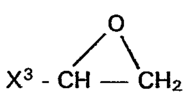 II

oder ein β-Ketohalogenid der Formel

$$X^3 - C(O) - CH_2 - Hal \qquad III$$

mit einem Amin der Formel

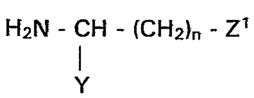 IV

oder

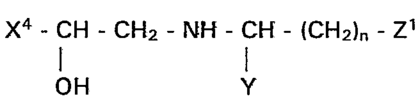 V

worin Hal Brom, Chlor oder Jod; eine der Gruppen $X^3$ und $X^4$ eine Gruppe $X^1$ und die andere eine Gruppe $X^2$ darstellt;

$Z^1$ einen Rest

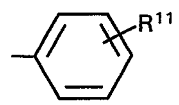 oder 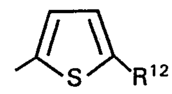 ($Z^1$)

$R^{11}$ und $R^{12}$ nieder-Alkanoyl, Carboxy, Cyano, Hydroxy-nieder-alkyl, Acyloxy oder einen Rest $-C(R^6)=C(R^7)COOR^8$, $-SO_2R^9$, $-C(O)R^9$ oder $-CH_2R$, nieder-Alkyl oder -Alkoxy ist;

und n, $X^1$, $X^2$, Y, R, $R^6$, $R^7$, $R^8$ und $R^9$ die bereits angegebene Bedeutung haben, umsetzt, wobei $X^3$ eine Gruppe $X^1$ ist, falls man eine Verbindung der Formel II oder III mit einer solchen der Formel IV umsetzt; dass man eine in einer erhaltenen Verbindung vorhandene $X^1$-C(O)- bzw. $X^2$-C(O)-Gruppe zu einer $X^1$-CHOH- bzw. $X^2$-CHOH-Gruppe reduziert und dass man gewünschtenfalls einen in einer Gruppe $X^1$, $X^2$, Y oder $Z^1$ des Reaktionsproduktes enthaltenen reaktionsfähigen Substituenten funktionell abwandelt.

**Patentansprüche für den Vertragsstaat:** AT

1. Verfahren zur Herstellung von Verbindungen der Formel

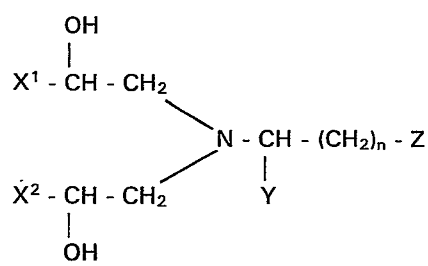 I

worin

n eine ganze Zahl von 1 bis 5;

$X^1$ Phenyl oder durch $R^1$, $R^2$ und $R^3$ mono-, di- oder tri-substituiertes Phenyl;

$X^2$ Wasserstoff, nieder-Alkyl, Phenyl oder durch $R^1$, $R^2$ und $R^3$ mono-, di- oder tri-substituiertes Phenyl;

$R^1$, $R^2$ und $R^3$ Wasserstoff, Halogen, Hydroxy, Benzyloxy, nieder-Alkyl, nieder-Alkoxy, Hydroxymethyl, Amino, Acylamino, nieder-Alkoxybenzylamino, Nitro, Carbamoyl, Trifluormethyl oder nieder-Alkylsulfonylmethyl;

Y Wasserstoff, nieder-Alkyl, Hydroxymethyl, Carboxy oder nieder-Alkoxycarbonyl;

Z ein Rest der Formel

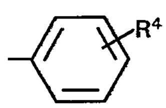 oder 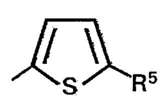 (Z)

$R^4$ und $R^5$ nieder-Alkanoyl, Carboxy, Cyano, Hydroxy-nieder-alkyl, Acyloxy oder einen Rest $-C(R^6)=C(R^7)COOR^8$, $-SO_2R^9$, $-C(O)R^9$ oder $-CH_2R^{10}$,

$R^6$, $R^7$ und $R^8$ Wasserstoff oder nieder-Alkyl;

$R^9$ Amino, mono-nieder-Alkylamino oder eine Gruppe R;

R di-nieder-Alkylamino, Piperidino, Morpholino, Thiamorpholino, Piperazino oder den Ätherrest eines niederaliphatischen, cycloaliphatischen oder araliphatischen Alkohols oder eines Phenols; und

$R^{10}$ eine Gruppe R darstellt und, falls $R^1$, $R^2$ und $R^3$ Wasserstoff, Halogen, Hydroxy, Benzyloxy, nieder-Alkyl, nieder-Alkoxy, Hydroxymethyl, Amino, nieder-Alkoxybenzylamino oder Trifluormethyl, und gleichzeitig Y Wasserstoff, nieder-Alkyl oder Hydroxymethyl sind, $R^{10}$ auch eine Gruppe Amino oder mono-nieder-Alkylamino darstellen kann,

sowie physiologisch verträgliche Salze davon, dadurch gekennzeichnet, dass man ein Epoxid der Formel

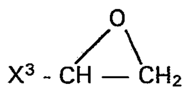 II

oder ein β-Ketohalogenid der Formel

$X^3 - C(O) - CH_2 - Hal$ III

mit einem Amin der Formel

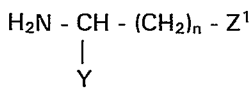 IV

oder

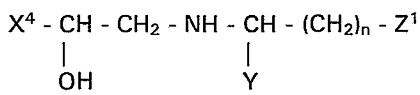 V

worin Hal Brom, Chlor oder Jod; eine der Gruppen $X^3$ und $X^4$ eine Gruppe $X^1$ und die andere eine Gruppe $X^2$ darstellt;

$Z^1$ einen Rest

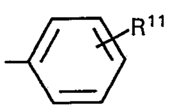 oder 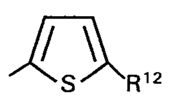 ($Z^1$)

$R^{11}$ und $R^{12}$ nieder-Alkanoyl, Carboxy, Cyano, Hydroxy-nieder-alkyl, Acyloxy oder einen Rest $-C(R^6)=C(R^7)COOR^8$, $-SO_2R^9$, $-C(O)R^9$ oder $-CH_2R$, und n, $X^1$, $X^2$, Y, R, $R^6$, $R^7$, $R^8$ und $R^9$ die bereits angegebene Bedeutung haben,

umsetzt, wobei $X^3$ eine Gruppe $X^1$ ist, falls man eine Verbindung der Formel II oder III mit einer solchen der Formel IV umsetzt; dass man eine in einer erhaltenen Verbindung vorhandene $X^1$-C(O)- bzw. $X^2$-C(O)-Gruppe zu einer $X^1$-CHOH- bzw. $X^2$-CHOH-Gruppe reduziert und dass man gewünschtenfalls einen in einer Gruppe $X^1$, $X^2$, Y oder $Z^1$ des Reaktionsproduktes enthaltenen reaktionsfähigen Substituenten funktionell abwandelt.

2. Verfahren nach Anspruch 1, dadurch gekennzeichnet, dass man eine Verbindung der Formel II mit einer solchen der Formel IV oder V umsetzt, worin $R^1$ Wasserstoff, $R^2$ und $R^3$ Wasserstoff, Halogen, Hydroxy, Benzyloxy, nieder-Alkyl, nieder-Alkoxy, Hydroxymethyl, Amino, Acylamino, nieder-Alkylbenzylamino, Nitro, Trifluormethyl oder nieder-Alkylsulfonylmethyl; $R^4$ und $R^5$ Carboxy, Cyano, Hydroxy-nieder-alkyl, Acyloxy oder einen Rest $-C(R^6)=C(R^7)COOR^8$, $-SO_2R^9$, $-C(O)R^9$ oder $-CH_2R^{10}$ sind.

3. Verfahren nach Anspruch 1 oder 2, dadurch gekennzeichnet, dass man Verbindungen herstellt, in denen n 1 oder 2, insbesondere 2, ist.

4. Verfahren nach Anspruch 1, 2 oder 3, dadurch gekennzeichnet, dass man Verbindungen herstellt, in denen $X^1$ Phenyl oder m-Trifluormethyl-phenyl, insbesondere Phenyl, ist.

5. Verfahren nach den Ansprüchen 1-4, dadurch gekennzeichnet, dass man Verbindungen herstellt, in denen $X^2$ Phenyl, m-Trifluormethyl-phenyl, nieder-Alkyl oder Wasserstoff, insbesondere Phenyl, ist.

6. Verfahren nach den Ansprüchen 1-5, dadurch gekennzeichnet, dass man Verbindungen herstellt, in denen Y Wasserstoff oder nieder-Alkyl, insbesondere Methyl, ist.

7. Verfahren nach den Ansprüchen 1 oder 3-6, dadurch gekennzeichnet, dass man Verbindungen herstellt, in denen $R^4$ und $R^5$ nieder-Alkanoyl, Carbamoyl, Sulfamoyl, nieder-Alkoxycarbonyl oder nieder-Alkylcarbamoyl, insbesondere nieder-Alkanoyl oder Carbamoyl, sind.

8. Verfahren nach den Ansprüchen 1 oder 3-7, dadurch gekennzeichnet, dass man Verbindungen herstellt, in denen n 1 oder 2, insbesondere 2; $X^1$ Phenyl oder m-Trifluormethyl-phenyl, insbesondere Phenyl; $X^2$ Phenyl, m-Trifluormethyl-phenyl, nieder-Alkyl oder Wasserstoff, insbesondere Phenyl; Y Wasserstoff oder nieder-Alkyl, insbesondere Methyl; $R^4$ und $R^5$ nieder-Alkanoyl, Carbamoyl, Sulfamoyl, nieder-Alkoxycarbonyl oder nieder-Alkylcarbamoyl, insbesondere nieder-Alkanoyl oder Carbamoyl, sind.

9. Verfahren nach den Ansprüchen 1-8, dadurch gekennzeichnet, dass man das p-[(R)-3-[Bis-[(R)-β-hydroxyphenäthyl]amino]butyl]benzamid herstellt.

**Claims for the Contracting States:**
BE, CH, DE, FR, GB, IT, LI, LU, NL, SE

1. Compounds of the formula

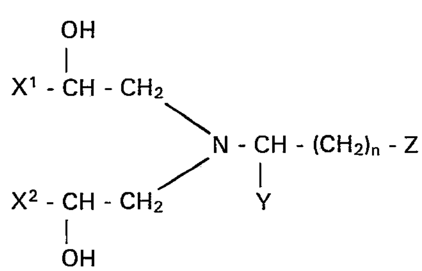 I

wherein

n represents a whole number of 1 to 5;

$X^1$ represents phenyl or phenyl mono-, di- or tri-substituted by $R^1$, $R^2$ and $R^3$;

$X^2$ represents hydrogen, lower-alkyl, phenyl or phenyl mono-, di- or tri-substituted by $R^1$, $R^2$ and $R^3$,

$R^1$, $R^2$ and $R^3$ represent hydrogen, halogen, hydroxy, benzyloxy, lower-alkyl, lower-alkoxy, hydroxymethyl, amino, acylamino, lower-alkoxybenzylamino, nitro, carbamoyl, trifluoromethyl or lower-alkylsulphonylmethyl;

Y represents hydrogen, lower-alkyl, hydroxymethyl, carboxy or lower-alkoxycarbonyl;

Z represents a residue of the formula

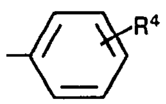 or 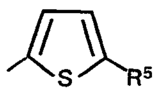 (Z)

$R^4$ and $R^5$ represent lower-alkanoyl, carboxy, cyano, hydroxy-lower-alkyl, acyloxy or a residue $-C(R^6)=C(R^7)COOR^8$, $-SO_2R^9$, $-C(O)R^9$ or $-CH_2R^{10}$,

$R^6$, $R^7$ and $R^8$ represent hydrogen or lower-alkyl;

$R^9$ represents amino, mono-lower-alkylamino or a group R;

R represents di-lower-alkylamino, piperidino, morpholino, thiamorpholino, piperazino or the ether residue of a lower aliphatic, cycloaliphatic or araliphatic alcohol or of a phenol; and

$R^{10}$ represents a group R and, where $R^1$, $R^2$ and $R^3$ are hydrogen, halogen, hydroxy, benzyloxy, lower-alkyl, lower-alkoxy, hydroxymethyl, amino, lower-alkoxybenzylamino or trifluoromethyl and simultaneously Y is hydrogen, lower-alkyl or hydroxymethyl, $R^{10}$ can also represent a group amino or mono-lower-alkylamino,

as well as physiologically compatible salts thereof.

2. Compounds in accordance with claim 1, in which $R^1$ is hydrogen, $R^2$ and $R^3$ are hydrogen, halogen, hydroxy, benzyloxy, lower-alkyl, lower-alkoxy, hydroxymethyl, amino, acylamino, lower-alkylbenzylamino, nitro, trifluoromethyl or lower-alkylsulphonylmethyl; $R^4$ and $R^5$ are carboxy, cyano, hydroxy-lower-alkyl, acyloxy, or a residue $-C(R^6)=C(R^7)COOR^8$, $-SO_2R^9$, $-C(O)R^9$ or $-CH_2R^{10}$.

3. Compounds in accordance with claim 1 or 2, in which n is 1 or 2, especially 2.

4. Compounds in accordance with claim 1, 2 or 3, in which $X^1$ is phenyl or m-trifluoromethyl-phenyl, especially phenyl.

5. Compounds in accordance with claims 1-4, in which $X^2$ is phenyl, m-trifluoromethyl, lower-alkyl or hydrogen, especially phenyl.

6. Compounds in accordance with claims 1-5, in which Y is hydrogen or lower-alkyl, especially methyl.

7. Compounds in accordance with claims 1 or 3-6, in which $R^4$ and $R^5$ are lower-alkanoyl, carbamoyl, sulphamoyl, lower-alkoxycarbonyl or lower-alkylcarbamoyl, especially lower-alkanoyl or carbamoyl.

8. Compounds in accordance with claims 1 or 3-7, in which n is 1 or 2, especially 2; $X^1$ is phenyl or m-trifluoromethylphenyl, especially phenyl; $X^2$ is phenyl, m-trifluoromethyl-phenyl, lower-alkyl or hydrogen, especially phenyl; Y is hydrogen or lower-alkyl, especially methyl; $R^4$ and $R^5$ are lower-alkanoyl, carbamoyl, sulphamoyl, lower-alkoxycarbonyl, or lower-alkylcarbamoyl, especially lower-alkanoyl or carbamoyl.

9. p-[(R)-3-[Bis-[(R)-β-hydroxyphenyethyl]amino]butyl]benzamide.

10. Compounds of formula I and physiologically compatible salts thereof as agents for the treatment of obesity, of diabetes mellitus and of conditions which are associated with an increased protein breakdown, or as feed additives for fattening animals.

11. Pharmaceutical preparations, characterized by a content of a compound of formula I or of a physiologically compatible salt thereof.

12. A process for the manufacture of compounds of formula I and of physiologically compatible salts thereof, characterized by reacting an epoxide of the formula

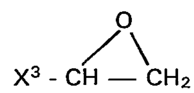 II

or a β-keto halide of the formula

$X^3 - C(O) - CH_2 - Hal$ III

with an amine of the formula

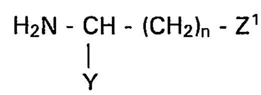 IV

or

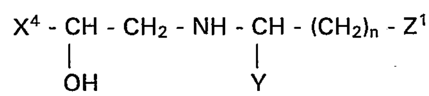 V

wherein Hal represents bromine, chlorine or iodine; one of the groups $X^3$ and $X^4$ represents a group $X^1$ and the other represents a group $X^2$;

$Z^1$ is a residue

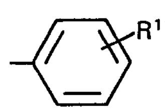 or 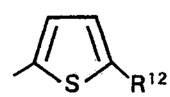 ($Z^1$)

$R^{11}$ and $R^{12}$ are lower-alkanoyl, carboxy, cyano, hydroxy-lower-alkyl, acyloxy or a residue $-C(R^6)=C(R^7)COOR^8$, $SO_2R^9$, $-C(O)R^9$ or $-CH_2R$, lower-alkyl or -alkoxy;

and n, $X^1$, $X^2$, Y, R, $R^6$, $R^7$, $R^8$ and $R^9$ have the significance already given,

whereby $X^3$ is a group $X^1$ where a compound of formula II or III is reacted with a compound of formula IV; reducting a $X^1$-C(O)- or $X^2$-C(O)- group in a compound obtained to a $X^1$-CHOH- or $X^2$-CHOH- group and, if desired, functionally modifying reactive substituents contained in a group $X^1$, $X^2$, Y or $Z^1$ of the reaction product.

**Claims for the Contracting State:** AT

1. A process for the manufacture of compounds of the formula

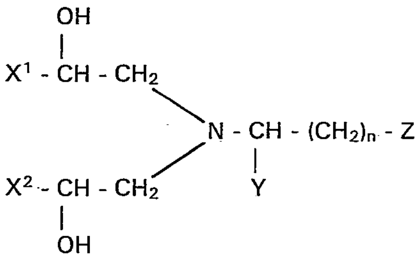

I

wherein

n represents a whole number of 1 to 5;

$X^1$ represents phenyl or phenyl mono-, di- or tri-substituted by $R^1$, $R^2$ and $R^3$;

$X^2$ represents hydrogen, lower-alkyl, phenyl or phenyl mono-, di- or tri-substituted by $R^1$, $R^2$ and $R^3$,

$R^1$, $R^2$ and $R^3$ represent hydrogen, halogen, hydroxy, benzyloxy, lower-alkyl, lower-alkoxy, hydroxymethyl, amino, acylamino, lower-alkoxybenzylamino, nitro, carbamoyl, trifluoromethyl or lower-alkylsulphonylmethyl;

Y represents hydrogen, lower-alkyl, hydroxymethyl, carboxy or lower-alkoxycarbonyl;

Z represents a residue of the formula

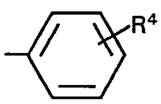 or 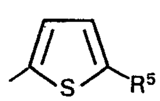 (Z)

$R^4$ and $R^5$ represent lower-alkanoyl, carboxy, cyano, hydroxy-lower-alkyl, acyloxy or a residue $-C(R^6)=C(R^7)COOR^8$, $-SO_2R^9$, $-C(O)R^9$ or $-CH_2R^{10}$,

$R^6$, $R^7$ and $R^8$ represent hydrogen or lower-alkyl;

$R^9$ represents amino, mono-lower-alkylamino or a group R;

R represents di-lower-alkylamino, piperidino, morpholino, thiamorpholino, piperazino or the ether residue of a lower aliphatic, cycloaliphatic or araliphatic alcohol or of a phenol; and

$R^{10}$ represents a group R and, where $R^1$, $R^2$ and $R^3$ are hydrogen, halogen, hydroxy, benzyloxy, lower-alkyl, lower-alkoxy, hydroxymethyl, amino, lower-alkoxybenzylamino or trifluoromethyl and simultaneously Y is hydrogen, lower-alkyl or hydroxymethyl, $R^{10}$ can also represent a group amino or mono-lower-alkylamino,

and of physiologically compatible salts thereof, characterized by reacting an epoxide of the formula

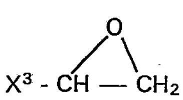

II

or a β-keto halide of the formula

$$X^3 - C(O) - CH_2 - Hal \qquad III$$

with an amine of the formula

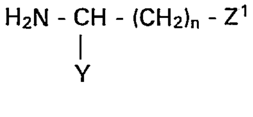

IV

or

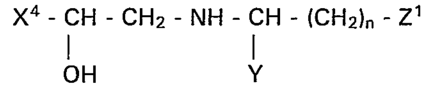

V

wherein Hal represents bromine, chlorine or iodine; one of the groups $X^3$ and $X^4$ represents a group $X^1$ and the other represents a group $X^2$;

$Z^1$ is a residue

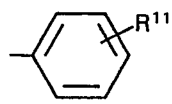 or 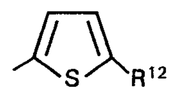 ($Z^1$)

$R^{11}$ and $R^{12}$ are lower-alkanoyl, carboxy, cyano, hydroxy-lower-alkyl, acyloxy or a residue $-C(R^6)=C(R^7)COOR^8$, $SO_2R^9$, $-C(O)R^9$ or $-CH_2R$,

and n, $X^1$, $X^2$, Y, R, $R^6$, $R^7$, $R^8$ and $R^9$ have the significance already given,

whereby $X^3$ is a group $X^1$ where a compound of formula II or III is reacted with a compound of formula IV; reducing a $X^1$-C(O)- or $X^2$-C(O)- group in a compound obtained to a $X^1$-CHOH- or $X^2$-CHOH- group and, if desired, functionally modifying reactive substituents contained in a group $X^1$, $X^2$, Y or $Z^1$ of the reaction product.

2. A process according to claim 1, characterized in that a compound of formula II is reacted with a compound of formula IV or V in which $R^1$ is hydrogen, $R^2$ and $R^3$ are hydrogen, halogen, hydroxy, benzyloxy, lower-alkyl, lower-alkoxy, hydroxymethyl, amino, acylamino, lower-alkylbenzylamino, nitro, trifluoromethyl or lower-alkylsulphonylmethyl; $R^4$ and $R^5$ are carboxy, cyano, hydroxy-lower-alkyl, acyloxy or a residue $-C(R^6)=C(R^7)COOR^8$, $-SO_2R^9$, $-C(O)R^9$ or $-CH_2R^{10}$.

3. A process according to claim 1 or 2, characterized in that compounds in which n is 1 or 2, especially 2, are manufactured.

4. A process according to claim 1, 2 or 3, characterized in that compounds in which $X^1$ is phenyl or m-trifluoromethyl-phenyl, especially phenyl, are manufactured.

5. A process according to claims 1-4, characterized in that compounds in which $X^2$ is phenyl, m--trifluoromethyl-phenyl, lower-alkyl or hydrogen, especially phenyl, are manufactured.

6. A process according to claims 1-5, characterized in that compounds in which Y is hydrogen or lower-alkyl, especially methyl, are manufactured.

7. A process according to claims 1 or 3-6, characterized in that compounds in which $R^4$ and $R^5$ are lower-alkanoyl, carbamoyl, sulphamoyl, lower-alkoxycarbonyl or lower-alkylcarbamoyl, especially lower-alkanoyl or carbamoyl, are manufactured.

8. A process according to claims 1 or 3-7, characterized in that compounds in which n is 1 or 2, especially 2; $X^1$ is phenyl or m-trifluoromethyl-phenyl, especially phenyl; $X^2$ is phenyl, m-trifluoromethyl-phenyl, lower-alkyl or hydrogen, especially phenyl; Y is hydrogen or lower-alkyl, especially methyl; $R^4$ and $R^5$ are lower-alkanoyl, carbamoyl, sulphamoyl, lower-alkoxycarbonyl or lower-alkylcarbamoyl, especially lower-alkanoyl or carbamoyl, are manufactured.

9. A process according to claims 1-8, characterized in that p-[(R)-3-[bis-[(R)-β-hydroxyphenethyl]-amino]butyl]benzamide is manufactured.

**Revendications pour les Etats contractants:**
BE, CH, DE, FR, GB, IT, LI, LU, NL, SE

1. Composés de formule

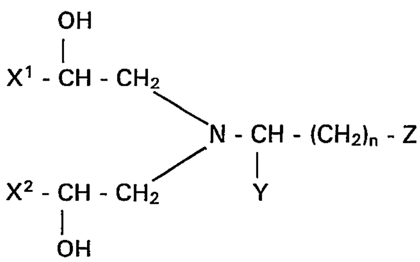

I

dans laquelle n est un nombre entier de 1 à 5;

$X^1$ est le phényle ou un phényle mono-, di- ou tri-substitué par $R^1$, $R^2$ et $R^3$;

$X^2$ est l'hydrogène, un alkyle inférieur, un phényle ou un phényle mono-, di- ou tri-substitué par $R^1$, $R^2$ et $R^3$,

$R^1$, $R^2$ et $R^3$ sont l'hydrogène, un halogène, hydroxy, benzyloxy, alkyle inférieur, alcoxy inférieur, hydroxyméthyle, amino, acylamino, alcoxybenzylamino inférieur, nitro, carbamoyle, trifluorométhyle ou alkylsulfonylméthyle inférieur;

Y est l'hydrogène, un alkyle inférieur, hydroxyméthyle, carboxy ou alcoxycarbonyle inférieur;

Z est un reste de formule

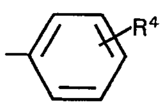 ou 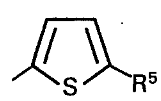 (Z)

$R^4$ et $R^5$ sont un alcanoyle inférieur, carboxy, cyano, hydroxyalkyle inférieur, acyloxy ou un reste $-C(R^6)=C(R^7)COOR^8$, $-SO_2R^9$, $-C(O)R^9$ ou $CH_2R^{10}$,

$R^6$, $R^7$ et $R^8$ sont l'hydrogène ou un alkyle inférieur;

$R^9$ est un amino, monoalkylamino inférieur ou un groupe R;

R est un dialkylamino inférieur, pipéridino, morpholino, thiamorpholino, pipérazino ou le reste éthéré d'un alcool aliphatique inférieur, cycloaliphatique ou araliphatique ou d'un phénol; et

$R^{10}$ représente un groupe R et si $R^1$, $R^2$ et $R^3$ sont l'hydrogène, un halogène, hydroxy, benzyloxy, alkyle inférieur, alcoxy inférieur, hydroxyméthyle, amino, alcoxybenzylamino inférieur ou trifluorométhyle, et qu'en même temps Y est l'hydrogène, un alkyle inférieur ou hydroxyméthyle, $R^{10}$ peut représenter aussi un groupe amino ou un monoalkylamino inférieur, ainsi que leurs sels physiologiquement compatibles.

2. Composés selon la revendication 1, dans lesquels $R^1$ est l'hydrogène, $R^2$ et $R^3$ sont l'hydrogène, un halogène, hydroxy, benzyloxy, alkyle inférieur, alcoxy inférieur, hydroxyméthyle, amino, acylamino, alkylbenzylamino inférieur, nitro, trifluorométhyle ou alkylsulfonyle méthyle inférieur; $R^4$ et $R^5$ sont un carboxy, cyano, hydroxyalkyle inférieur, acyloxy ou un reste $-C(R^6)=C(R^7)COOR^8$, $-SO_2R^9$, $-C(O)R^9$ ou $-CH_2R^{10}$.

3. Composés selon la revendication 1 ou 2, dans lesquels n est 1 ou 2, en particulier 2.

4. Composés selon la revendication 1, 2 ou 3, dans lesquels $X^1$ est un phényle ou m-trifluorométhylphényle, en particulier un phényle.

5. Composés selon les revendications 1-4, dans lesquels $X^2$ est un phényle, m-trifluorométhylphényle, alkyle inférieur ou hydrogène, en particulier un phényle.

6. Composés selon les revendications 1-5, dans lesquels Y est l'hydrogène ou un alkyle inférieur, en particulier un méthyle.

7. Composés selon les revendications 1 ou 3-6, dans lesquels $R^4$ et $R^5$ sont un alcanoyle inférieur, carbamoyle, sulfamoyle, alcoxycarbonyle inférieur ou alkylcarbamoyle inférieur, en particulier un alcanoyl inférieur ou carbamoyle.

8. Composés selon les revendications 1 ou 3-7, dans lesquels n est 1 ou 2, en particulier 2; $X^1$ est un phényle ou m-trifluorométhylphényle, en particulier phényle; $X^2$ est un phényle, m-trifluorométhylphényle, alkyle inférieur ou hydrogène, en particulier phényle; Y est l'hydrogène ou un alkyle inférieur, en particulier méthyle; $R^4$ et $R^5$ sont un alcanoyle inférieur, carbamoyle, sulfamoyle, alcoxycarbonyle inférieur, ou alkylcarbamoyle inférieur, en particulier alcanoyle inférieur ou carbamoyle.

9. p-[(R)-3-[bis-[(R)-β-hydroxyphényéthyl]amino]butyl]benzamide.

10. Composés de formule I et leurs sels physiologiquement compatibles comme moyens de traitement de l'adipose, du diabète sucré et des états qui sont liés à un taux élevé de dégradation de protéines, ou comme additif alimentaire pour animaux engraissés.

11. Préparations pharmaceutiques, caractérisées par une teneur en un composé de formule I ou en un de ses sels physiologiquement compatibles.

12. Procédé de préparation de composés de formule I et de ses sels physiologiquement compatibles, caractérisé en ce qu'on fait réagir un époxyde de formule

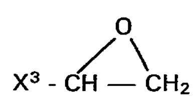

II

ou un β-cétohalogénure de formule

$X^3 - C(O) - CH_2 - Hal$ III

avec une amine de formule

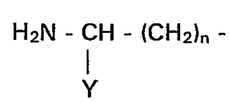

IV

ou

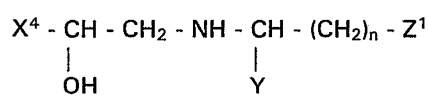

V

dans laquelle Hal est le brome, le chlore ou l'iode; l'un des groupes $X^3$ et $X^4$ représente un groupe $X^1$ et l'autre un groupe $X^2$;

$Z^1$ est un reste

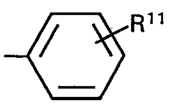
ou
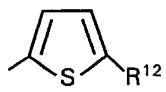
($Z^1$)

$R^{11}$ et $R^{12}$ sont un alcanoyle inférieur, carboxy, cyano, hydroxyalkyle inférieur, acyloxy ou un reste $-C(R^6)=C(R^7)COOR^8$, $SO_2R^9$, $-C(O)R^9$ ou $-CH_2R$, alkyle inférieur ou alcoxy inférieur;

et n, $X^1$, $X^2$, Y, R, $R^6$, $R^7$, $R^8$ et $R^9$ ont les significations déjà données,

dans lesquelles $X^3$ est un groupe $X^1$, si l'on fait réagir un composé de formule II ou III avec un même de formule IV, on réduit un groupe existant $X^1$-C(O)- ou $X^2$-C(O)- dans un composé obtenu en un groupe $X^1$-CHOH- ou $X^2$-CHOH- et on transforme fonctionnellement, si on le souhaite un substituant pouvant réagir contenu dans un groupe $X^1$, $X^2$, Y ou $Z^1$ du produit de la réaction.

**Revendications pour l'Etat contractant:** AT

1. Procédé de préparation de composés de formule

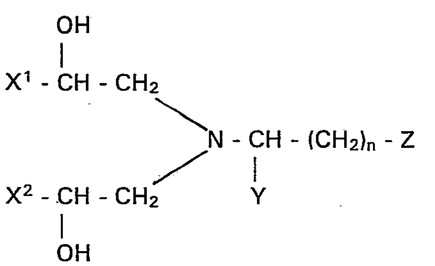
I

dans laquelle n est un nombre entier de 1 à 5;

$X^1$ est le phényle ou un phényle mono-, di- ou tri-substitué par $R^1$, $R^2$ et $R^3$;

$X^2$ est l'hydrogène, un alkyle inférieur, un phényle ou un phényle mono-, di- ou tri-substitué par $R^1$, $R^2$ et $R^3$,

$R^1$, $R^2$ et $R^3$ sont l'hydrogène, un halogène, hydroxy, benzyloxy, alkyle inférieur, alcoxy inférieur, hydroxyméthyle, amino, acylamino, alcoxybenzylamino inférieur, nitro, carbamoyle, trifluorométhyle ou alkylsulfonylméthyle inférieur;

Y est l'hydrogène, un alkyle inférieur, hydroxyméthyle, carboxy ou alcoxycarbonyle inférieur;

Z est un reste de formule

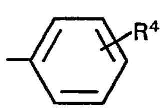
ou
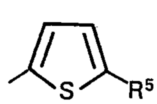
(Z)

$R^4$ et $R^5$ sont un alcanoyle inférieur, carboxy, cyano, hydroxyalkyle inférieur, acyloxy ou un reste $-C(R^6)=C(R^7)COOR^8$, $-SO_2R^9$, $-C(O)R^9$ ou $CH_2R^{10}$,

$R^6$, $R^7$ et $R^8$ sont l'hydrogène ou un alkyle inférieur;

$R^9$ est un amino, monoalkylamino inférieur ou un groupe R;

R est un dialkylamino inférieur, pipéridino, morpholino, thiamorpholino, pipérazino ou le reste éthéré d'un alcool aliphatique inférieur, cycloaliphatique ou araliphatique ou d'un phénol; et

$R^{10}$ représente un groupe R et si $R^1$, $R^2$ et $R^3$ sont l'hydrogène, un halogène, hydroxy, benzyloxy, alkyle inférieur, alcoxy inférieur, hydroxyméthyle, amino, alcoxybenzylamino inférieur ou trifluorométhyle, et qu'en même temps Y est l'hydrogène, un alkyle inférieur ou hydroxy méthyle, $R^{10}$ peut représenter aussi un groupe amino ou un monoalkylamino inférieur, ainsi que leurs sels physiologiquement compatibles,

caractérisé en ce qu'on fait réagir un composé de formule

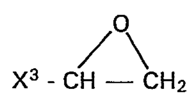
II

ou un β-cétohalogénure de formule

$X^3 - C(O) - CH_2 - Hal$ III

avec une amine de formule

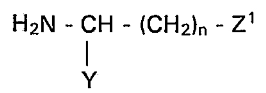
IV

ou

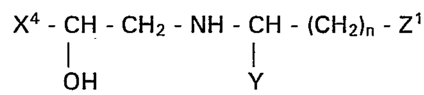
V

dans laquelle Hal est le brome, le chlore ou l'iode; l'un des groupes $X^3$ et $X^4$ représente un groupe $X^1$ et l'autre un groupe $X^2$;

$Z^1$ est un reste

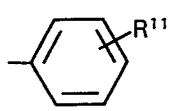
ou
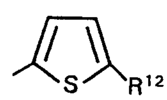
($Z^1$)

$R^{11}$ et $R^{12}$ sont un alcanoyl inférieur, carboxy, cyano, hydroxyalkyle inférieur, acyloxy ou un reste $-C(R^6)=C(R^7)COOR^8$, $SO_2R^9$, $-C(O)R^9$ ou $-CH_2R$,

et n, $X^1$, $X^2$, Y, R, $R^6$, $R^7$, $R^8$ et $R^9$ ont les significations déjà données,

dans lesquelles $X^3$ est un groupe $X^1$, si l'on fait réagir un composé de formule II ou III avec un même de formule IV; on réduit un groupe existant $X^1$-C(O)- ou $X^2$-C(O)- dans un composé obtenu en un groupe $X^1$-CHOH- ou $X^2$-CHOH- et on transforme fonctionnellement, si on le souhaite un substituant pouvant réagir contenu dans un groupe $X^1$, $X^2$, Y ou $Z^1$ du produit de la réaction.

2. Procédé selon la revendication 1, caractérisé

en ce qu'on fait réagir un composé de formule II avec un même de formule IV ou V, dans lesquels $R^1$ est l'hydrogène, $R^2$ et $R^3$ sont l'hydrogène, un halogène, hydroxy, benzyloxy, alkyle inférieur, alcoxy inférieur, hydroxyméthyle, amino, acylamino, alkylbenzylamino inférieur, nitro, trifluorométhyle ou alkylsulfonylméthyle inférieur; $R^4$ et $R^5$ sont un carboxy, cyano, hydroxyalkyle inférieur, acyloxy ou un reste $-C(R^6)=C(R^7)COOR^8$, $-SO_2R^9$, $-C(O)R^9$ ou $-CH_2R^{10}$.

3. Procédé selon la revendication 1 ou 2, caractérisé en ce qu'on prépare des composés, dans lesquels n est 1 ou 2, en particulier 2.

4. Procédé selon la revendication 1, 2 ou 3, caractérisé en ce qu'on prépare des composés dans lesquels $X^1$ est un phényle ou m-trifluorométhylphényle, en particulier un phényle.

5. Procédé selon les revendications 1-4, caractérisé en ce qu'on prépare des composés dans lesquels $X^2$ est un phényle, m-trifluorométhylphényle, alkyle inférieur ou hydrogène, en particulier un phényle.

6. Procédé selon les revendications 1-5 caractérisé en ce qu'on prépare des composés dans lesquels Y est l'hydrogène ou un alkyle inférieur, en particulier un méthyle.

7. Procédé selon les revendications 1 ou 3-6, caractérisé en ce qu'on prépare des composés dans lesquels $R^4$ et $R^5$ sont un alcanoyle inférieur, carbamoyle, sulfamoyle, alcoxycarbonyle inférieur ou alkylcarbamoyle inférieur, en particulier un alcanoyle inférieur ou carbamoyle.

8. Procédé selon les revendications 1 ou 3-7, caractérisé en ce qu'on prépare des composés dans lesquels n est 1 ou 2, en particulier 2; $X^1$ est un phényle ou m-trifluorométhylphényle, en particulier phényle; $X^2$ est un phényle, m-trifluorométhylphényle, alkyle inférieur ou hydrogène, en particulier phényle; Y est l'hydrogène ou un alkyle inférieur, en particulier méthyle; $R^4$ et $R^5$ sont un alcanoyle inférieur, carbamoyle, sulfamoyle, alcoxycarbonyle inférieur, ou alkylcarbamoyle inférieur, en particulier alcanoyle inférieur ou carbamoyle.

9. Procédé selon les revendications 1-8, caractérisé en ce qu'on prépare le p-[(R)-3-[bis-[(R)-β-hydroxyphényéthyl]amino]butyl]benzamide.